# EUROPEAN PATENT APPLICATION

(11) **EP 0 675 127 A1**
(43) Date of publication of application: **04.10.1995**
(21) Application number: 94903073.8
(22) Date of filing: 27.12.1993
(51) Int. Cl.: C07D 513/04, C07D 215/56, A61K 31/47

(54) **QUINOLINECARBOXYLIC ACID DERIVATIVE AND PROCESS FOR PRODUCING THE SAME**

(30) Priority: 28.12.1992 JP 360552/92; 01.10.1993 JP 270059/93
(71) Applicant: NIPPON SHINYAKU COMPANY, LIMITED, Minami-ku Kyoto-shi Kyoto 601 (JP)
(72) Inventor: SEGAWA, Jun, Nara-shi, Nara 631 (JP); MATSUOKA, Masato, Kyoto-shi, Kyoto 606 (JP); TOMII, Yoshifumi, Katano-shi, Osaka 576 (JP)
(74) Representative: Hillier, Peter
(86) International application number: JP9301897
(87) International publication number: WO9414819

(57) **Abstract**

A quinolinecarboxylic acid derivative represented by general formula (I) and a pharmaceutically acceptable salt thereof, wherein z represents (un)substituted cyclic amino or phenyl; A represents > C=CH₂ or > CHR¹, R¹ representing flouroalkyl; R² represents hydrogen, alkyl, alkoxy, amino or halogen; and R³ represents hydrogen or alkyl. The invention compound is useful for treating various infectious diseases, in particular, those caused by Gram-positive bacteria including methicillin resistant *Staphylococcus aureus* and new quinolone and methicillin resistant resistant *Staphylococcus aureus*.

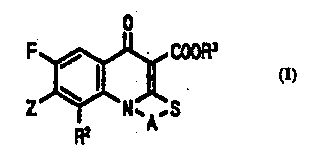

## Description

### TECHNICAL FIELD

The present invention relates to a novel quinolinecarboxylic acid derivative which has antibacterial activity and is of value as a therapeutic agent for various infectious diseases.

### BACKGROUND TECHNOLOGY

Synthetic antibacterial agents known as new quinolone drugs such as norfloxacin, enoxacin, ofloxacin, ciprofloxacin and tosufloxacin are being used broadly in the treatment of bacterial infections today.

While each of these synthetic antimicrobials has a broad antibacterial spectrum covering gram-negative and gram-positive bacteria, they are only sparingly active against methicillin-resistant Staphylococcus aureus and new quinolone-resistant and methicillin-resistant Staphylococcus aureus which are current foci of attention in regard to multiple drug resistance.

The therapeutic agent generally acknowledged to be effective in infections due to new quinolone-resistant and methicillin-resistant S. aureus is the antibiotic vancomycin. However, this antibiotic is highly nephrotoxic and, therefore, cannot necessarily be considered satisfactory.

As synthetic antibacterials in the quinolone series which is effective on new quinolone-resistant and methicillin-resistant S. aureus, DU-6859a and BAY Y 3118 are being developed but neither has reached the stage of clinical application yet.

The state of the art, therefore, is that no fully satisfactory therapeutic drug is available for the treatment of infections caused by methicillin-resistant S. aureus or new quinolone-resistant and methicillin-resistant S. aureus.

Meanwhile, 6-fluorothiazetoquinolinecarboxylic acid derivatives are disclosed in Japanese Patent (JP) Kokai No. S 63-107990, JP Kokai No. H 1-294680, JP Kokai No. H 1-230584, JP Kohyo No. 91/07412 and JP Kohyo No. 92/06099.

These disclosed compounds are not sufficiently active against methicillin-resistant S. aureus and/or new quinolone-resistant and methicillin-resistant S. aureus.

The present invention, therefore, has as its object to provide a novel quinolinecarboxylic acid derivative which is useful for the treatment of various infections, particularly infections associated with gram-positive bacteria including methicillin-resistant S. aureus and/or new quinolone-resistant and methicillin-resistant S. aureus.

It is a further object of the present invention to provide pivotal intermediates, which is used for the production of thiazetoquinolinecarboxylic acid derivatives having high therapeutic efficacy for infectious diseases and processes for producing optically active intermediates.

To accomplish the above objects, the inventors of the present invention synthesized and evaluated a variety of 6-fluorothiazetoquinolinecarboxylic acid derivatives that might be effective for various infections, and in the course of their research, found by chance that thiazetoquinolinecarboxylic acid derivatives having a methylene or fluoroalkyl group in lieu of 1-hydrogen have very potent antibacterial activity and constitute a group of compounds which is not only highly active against methicillin-resistant S. aureus and new quinolone-resistant and methicillin-resistance S. aureus for which no satisfactory antibacterial agents has heretofore been available but also effective in infections in animals and is of low toxicity potential. The present invention has been developed on the basis of the above findings.

### DISCLOSURE OF INVENTION

The present invention is directed to a novel thiazetoquinolinecarboxylic acid derivative which is useful for the treatment of various infectious diseases, particularly infectious diseases caused by gram-positive bacteria including methicillin-resistant Staphylococcus aureus (MRSA) and new quinolone-resistant and methicillin-resistant Staphylococcus aureus (MRSA, pCA^{r}).

More particularly, the present invention relates to a thiazetoquinolinecarboxylic acid derivative of the following general formula [I] and a pharmaceutically acceptable salt thereof. 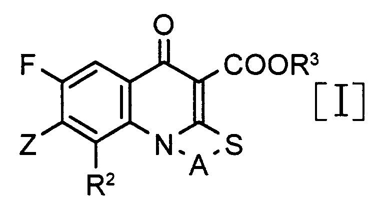
wherein Z represents substituted or
unsubstituted cyclic amino or phenyl.

A represents >C=CH₂ or >CHR¹ (R¹ : fluoroalkyl).
R² represents hydrogen, alkyl, alkoxy, amino, or halogen.
R³ represents hydrogen or alkyl.
[I] includes [Ib], [Ic] and [Id] which are described hereinafter.

The compound of the present invention is characterized in that the 1-position of the 6-fluorothiazetoquinolinecarboxylic acid derivative is substituted by methylene or fluoroalkyl.

The present invention relates, furthermore, to a compound of the following general formula [XII], which is a pivotal intermediate for the production of thiazetoquinolinecarboxylic acid derivatives which are of value for the treatment of infectious diseases. 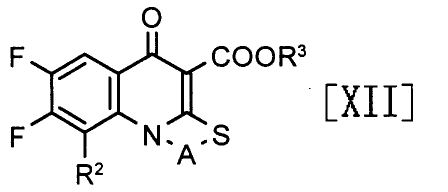
wherein A, R² and R³ are as defined above.

The present invention further relates to a compound of the following general formula [XI], which is another intermediate of importance. 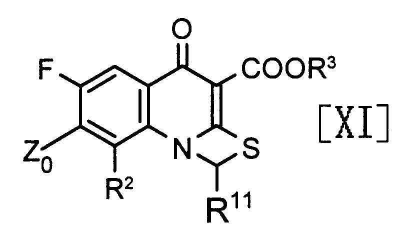
wherein Z₀ represents halogen, or substituted or unsubstituted cyclic amino or phenyl. R² and R³ are as defined above. R¹¹ represents hydroxyalkyl, acyloxyalkyl or alkoxyalkyl.

[XI] includes [XIe], [XIf], [XIg] and [XIk] which are described hereinafter. Similarly, [II] includes [IIe], [IIf] and [IIg].

In a further object, the present invention relates to a process for producing optically active intermediate compounds, which can be written as follows. 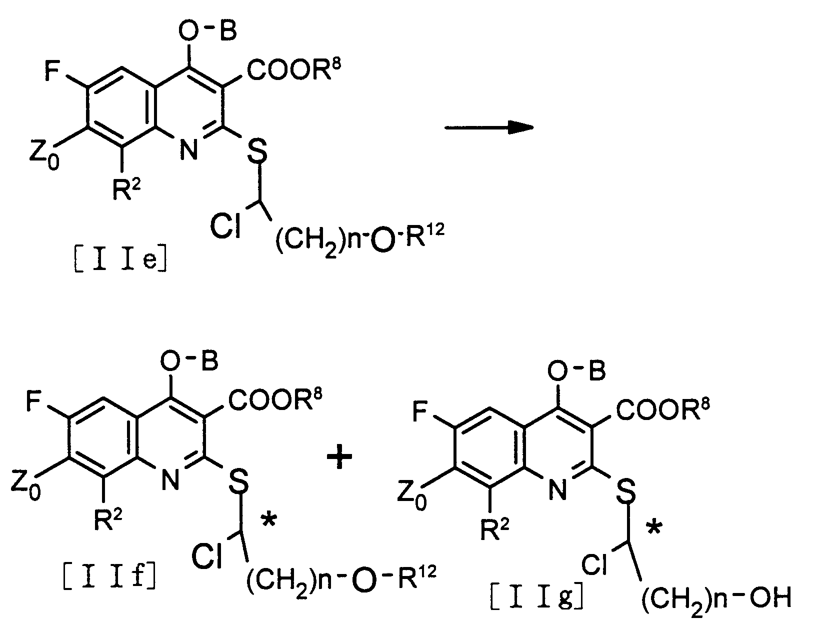
Thus, one of the inventions is concerned with a process for producing optically active quinolinecarboxylic acid derivatives represented by general formulas [IIf] and [IIg], respectively, characterized in that a substrate compound of general formula [IIe] is subjected to selective alcoholysis of one of its optical isomers in the presence of an enzyme having stereoselective transesterase activity followed by separation.

In the above formulas, Z₀ represents halogen, or substituted or unsubstituted cyclic amino or phenyl; R² represents hydrogen, alkyl, alkoxy, amino, or halogen; R⁸ represents alkyl; R¹² represents acyl; n represents an integer of 1-10; B represents a hydroxy-protecting group.

In still another aspect, the present invention relates to a process for producing optically active intermediate compounds, which can be shown in the following reaction scheme. 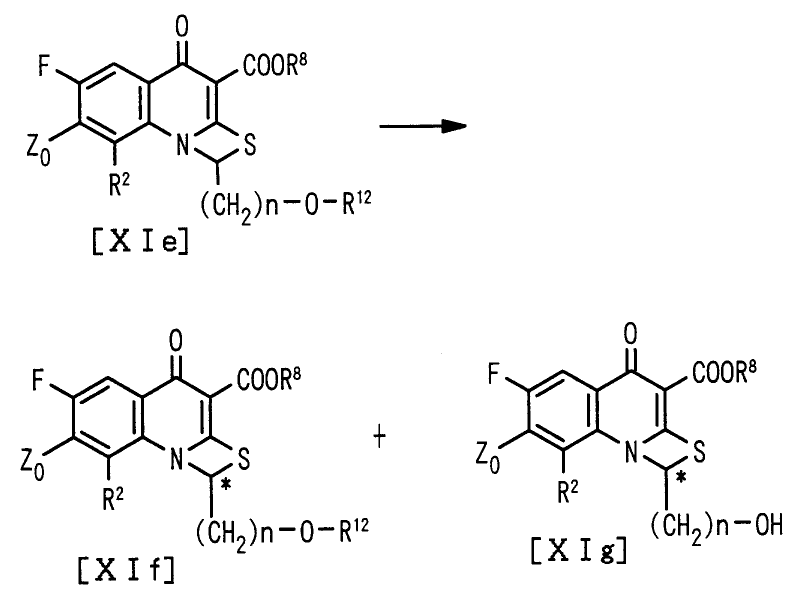
Thus, one of the inventions is directed to a process for producing optically active thiazetoquinolinecarboxylic acid derivatives represented by general formulas [XIf] and [XIg], respectively, characterized in that a substrate compound of general formula [XIe] is subjected to selective alcoholysis of one of its optical isomers in the presence of an enzyme having stereoselective transesterase activity followed by separation.

In the above formulas, Z₀ represents halogen, or substituted or unsubstituted cyclic amino or phenyl; R² represents hydrogen, alkyl, alkoxy, amino, or halogen; R⁸ represents alkyl; R¹² represents acyl; n represents an integer of 1-4.

In still another objecta, the present invention relates to a process for producing optically active intermediate compounds, which can be shown in the following reaction scheme. 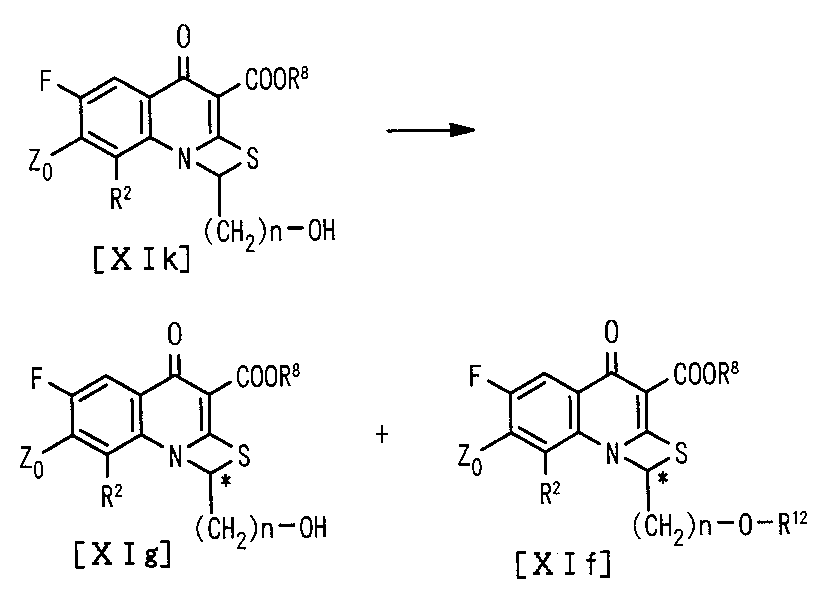
One of the inventions, therefore, is directed to a process for producing optically active thiazetoquinolinecarboxylic acid derivatives represented by general formulas [XIg] and [XIf], respectively, characterized in that a substrate compound of general formula [XIk] is esterified with an acyl donor in the presence of an enzyme having stereoselective transesterase activity followed by separation to give each of optical isomer.

In the above formulas, Z₀ represents halogen, or substituted or unsubstituted cyclic amino or phenyl; R² represents hydrogen, alkyl, alkoxy, amino, or halogen; R⁸ represents alkyl, R¹² represents acyl; n represents an integer of 1-4.

The present invention is now described in detail.

The said cyclic amino is a 4- through 8-membered cyclic amino group which may contain further nitrogen, oxygen or sulfur as the ring member or members. The cyclic amino as such includes but is not limited to azetidino, pyrrolidinyl, piperidino, azepinyl, azocino, piperazinyl, homopiperazinyl, pyrrolino, morpholino, thiomorpholino, 3,7-diazabicyclo[3.3.0]octa-1(5)-en-3-yl, 4,7-diazaspiro[2.5]octen-7-yl, 7-amino-5-azaspiro[2.4]heptan-5-yl and so on. The cyclic amino group may have one or more substituents which may for example be alkyl, hydroxyalkyl, aminoalkyl, acyl, amino, hydroxy, (5-methyl-2-oxo-1,3-dioxol-4-yl)methyl and/or the like. Particularly preferred are substituted or unsubstituted pyrrolidinyl and piperazinyl.

Substituent groups for said phenyl may be amino, alkylamino, aminoalkyl, hydroxy, alkoxy or acyl.

The fluoroalkyl includes but is not limited to fluoromethyl, 1-fluoroethyl, 2-fluoroethyl, 1-fluoropropyl, 2-fluoropropyl, 3-fluoropropyl, 1-fluorobutyl, 2-fluorobutyl, 3-fluorobutyl, 4-fluorobutyl, difluoromethyl, 2,2-difluoroethyl, 3,3-difluoropropyl, 4,4-difluorobutyl, trifluoromethyl, 2,2,2-trifluoroethyl, 3,3,3-trifluoropropyl, 4,4,4-trifluorobutyl and so on. Particularly preferred are 1-fluoromethyl and 1,1-difluoromethyl.

The alkyl is preferably a straight-chain or branched-chain alkyl group of 1-10 carbon atoms, thus including methyl, ethyl, n-propyl, isopropyl, n-butyl, isobutyl, sec-butyl, tert-butyl, n-amyl, isoamyl, n-hexyl, isohexyl, n-heptyl, isoheptyl, n-octyl, and isooctyl, among others.

The halogen includes fluorine, chlorine, bromine, and iodine.

The alkoxy is preferably a straight-chain or branched-chain lower alkoxy group of 1-4 carbon atoms, thus including methoxy, ethoxy, n-propoxy, isopropoxy, n-butoxy, isobutoxy, sec-butoxy, and tert-butoxy.

The acyl includes straight-chain or branched-chain alkanoyl groups of 1-4 carbon atoms, such as formyl, acetyl, propionyl, butyryl and trifluoroacetyl, and aroyl groups of 7-8 carbon atoms, such as benzoyl, and benzylcarbonyl.

The alkyl moiety of hydroxyalkyl, the alkyl and acyl moieties of acyloxyalkyl, and the alkyl and alkoxy moieties of alkoxyalkyl may all be exemplified by the respective groups mentioned above.

Where any of compounds [I], [XI] and [XII] of the present invention contains an asymmetric carbon atom, there exist optical isomers due to the carbon atom and these isomers and mixtures thereof are also subsumed in the concept of the present invention.

The salt of compound [I] as covered by the present invention includes salts with mineral acids such as hydrochloric acid, sulfuric acid, nitric acid, phosphoric acid, hydrofluoric acid and hydrobromic acid, salts with organic acids such as formic acid, acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid and camphorsulfonic acid, and salts with alkali metal or alkaline earth metal elements such as sodium, potassium and calcium.

The compound [I] of the present invention includes but is not limited to the following species in addition to the species mentioned in the production examples presented hereinafter.

6-Fluoro-1-methylene-4-oxo-7-(1-piperazinyl)-4H-[1,3]thiazeto[3,2-a]quinoline-3-carboxylic acid,
6,8-Difluoro-1-methylene-4-oxo-7-(1-piperazinyl)-4H-[1,3]thiazeto[3,2-a]quinoline-3-carboxylic acid,
6,8-Difluoro-1-fluoromethyl-4-oxo-7-(1-piperazinyl)-4H-[1,3]thiazeto[3,2-a]quinoline-3-carboxylic acid,
6-Fluoro-1-fluoromethyl-4-oxo-7-piperidino-4H-[1,3]thiazeto[3,2-a]quinoline-3-carboxylic acid,
6-Fluoro-1-fluoromethyl-4-oxo-7-(1-pyrrolidinyl)-4H-[1,3]thiazeto[3,2-a]quinoline-3-carboxylic acid,
6-Fluoro-7-(3,5-dimethyl-1-piperazinyl)-1-methylene-4-oxo-4H-[1,3]thiazeto[3,2-a]quinoline-3-carboxylic acid,
6-Fluoro-1-fluoromethyl-7-(3,5-dimethyl-1-piperazinyl)-4-oxo-4H-[1,3]thiazeto[3,2-a]quinoline-3-carboxylic acid,
6-Fluoro-1-methylene-4-oxo-7-thiomorpholino-4H-[1,3]thiazeto[3,2-a]quinoline-3-carboxylic acid,
7-(4-Aminopiperidino)-6-fluoro-1-methylene-4-oxo-4H-[1,3]thiazeto[3,2-a]quinoline-3-carboxylic acid,
7-[(S)-3-Amino-1-pyrrolidinyl]-6,8-difluoro-1-methylene-4-oxo-4H-[1,3]thiazeto[3,2-a]quinoline-3-carboxylic acid,
7-[(S)-3-Amino-1-pyrrolidinyl]-6,8-difluoro-1-fluoromethyl-4-oxo-4H-[1,3]thiazeto[3,2-a]quinoline-3-carboxylic acid,
7-[(S)-3-Amino-1-pyrrolidinyl]-8-chloro-6-fluoro-1-methylene-4-oxo-4H-[1,3]thiazeto[3,2-a]quinoline-3-carboxylic acid,
7-[(S)-3-Amino-1-pyrrolidinyl]-8-chloro-6-fluoro-1-fluoromethyl-4-oxo-4H-[1,3]thiazeto[3,2-a]quinoline-3-carboxylic acid,
7-[(R)-3-Amino-1-pyrrolidinyl]-6-fluoro-1-fluoromethyl-4-oxo-4H-[1,3]thiazeto[3,2-a]quinoline-3-carboxylic acid,
7-[(R)-3-Amino-1-pyrrolidinyl]-6,8-difluoro-1-methylene-4-oxo-4H-[1,3]thiazeto[3,2-a]quinoline-3-carboxylic acid,
7-[(R)-3-Amino-1-pyrrolidinyl]-6,8-difluoro-1-fluoromethyl-4-oxo-4H-[1,3]thiazeto[3,2-a]quinoline-3-carboxylic acid,
7-[(R)-3-Amino-1-pyrrolidinyl]-8-chloro-6-fluoro-1-methylene-4-oxo-4H-[1,3]thiazeto[3,2-a]quinoline-3- carboxylic acid,
7-[(R)-3-Amino-1-pyrrolidinyl]-8-chloro-6-fluoro-1-fluoromethyl-4-oxo-4H-[1,3]thiazeto[3,2-a]quinoline-3-carboxylic acid,
6-Fluoro-1-fluoromethyl-4-oxo-7-phenyl-4H-[1,3]thiazeto[3,2-a]quinoline-3-carboxylic acid,
7-(4-Aminophenyl)-6-fluoro-1-fluoromethyl-4-oxo-4H-[1,3]thiazeto[3,2-a]quinoline-3-carboxylic acid,
6,8-Difluoro-1-fluoromethyl-4-oxo-7-phenyl-4H-[1,3]thiazeto[3,2-a]quinoline-3-carboxylic acid,
6,8-Difluoro-1-methylene-4-oxo-7-phenyl-4H-[1,3]thiazeto[3,2-a]quinoline-3-carboxylic acid,
7-(4-Aminophenyl)-6,8-difluoro-1-fluoromethyl-4-oxo-4H-[1,3]thiazeto[3,2-a]quinoline-3-carboxylic acid,
7-(4-Aminophenyl)-6-fluoro-1-fluoromethyl-8-methoxy-4-oxo-4H-[1,3]thiazeto[3,2-a]quinoline-3-carboxylic acid,
6-Fluoro-1-fluoromethyl-8-methoxy-4-oxo-7-phenyl-4H-[1,3]thiazeto[3,2-a]quinoline-3-carboxylic acid,
6-Fluoro-1-methylene-8-methoxy-4-oxo-7-phenyl-4H-[1,3]thiazeto[3,2-a]quinoline-3-carboxylic acid,
8-Chloro-6-fluoro-1-fluoromethyl-4-oxo-7-phenyl-4H-[1,3]thiazeto[3,2-a]quinoline-3-carboxylic acid,
8-Chloro-6-fluoro-1-methylene-4-oxo-7-phenyl-4H-[1,3]thiazeto[3,2-a]quinoline-3-carboxylic acid,
7-(4-Aminophenyl)-8-chloro-6-fluoro-1-fluoromethyl-4-oxo-4H-[1,3]thiazeto[3,2-a]quinoline-3-carboxylic acid,
7-(4-Aminophenyl)-8-chloro-6-fluoro-1-methylene-4-oxo-4H-[1,3]thiazeto[3,2-a]quinoline-3-carboxylic acid,
7-(4-Aminomethylphenyl)-8-chloro-6-fluoro-1-fluoromethyl-4-oxo-4H-[1,3]thiazeto[3,2-a]quinoline-3-carboxylic acid,
7-(4-Aminomethylphenyl)-8-chloro-6-fluoro-1-methylene-4-oxo-4H-[1,3]thiazeto[3,2-a]quinoline-3-carboxylic acid,
8-Chloro-6-fluoro-1-fluoromethyl-7-(4-methoxyphenyl)-4-oxo-4H-[1,3]thiazeto[3,2-a]quinoline-3-carboxylic acid,
8-Chloro-6-fluoro-1-methylene-7-(4-methoxyphenyl)-4-oxo-4H-[1,3]thiazeto[3,2-a]quinoline-3-carboxylic acid,
8-Chloro-6-fluoro-1-fluoromethyl-7-(4-hydroxyphenyl)-4-oxo-4H-[1,3]thiazeto[3,2-a]quinoline-3-carboxylic acid,
8-Chloro-6-fluoro-7-(4-hydroxyphenyl)-1-methylene-4-oxo-4H-[1,3]thiazeto[3,2-a]quinoline-3-carboxylic acid,
Ethyl 7-(3-amino-1-pyrrolidinyl)-6-fluoro-1-fluoromethyl-4-oxo-4H-[1,3]thiazeto[3,2-a]quinoline-3-carboxylate,
7-(3-Amino-1-azetidinyl)-6-fluoro-1-fluoromethyl-4-oxo-4H-[1,3]thiazeto[3,2-a]quinoline-3-carboxylic acid,
7-(3-Amino-3-methyl-1-azetidinyl)-6-fluoro-1-fluoromethyl-4-oxo-4H-[1,3]thiazeto[3,2-a]quinoline-3-carboxylic acid,
7-(3-Aminomethyl-1-azetidinyl)-6-fluoro-1-fluoromethyl-4-oxo-4H-[1,3]thiazeto[3,2-a]quinoline-3-carboxylic acid and
7-(3-Aminomethyl-3-methyl-1-azetidinyl)-6-fluoro-1-fluoromethyl-4-oxo-4H-[1,3]thiazeto[3,2-a]quinoline-3-carboxylic acid.

The compound [I] of the present invention can be produced, for example, the following processes. 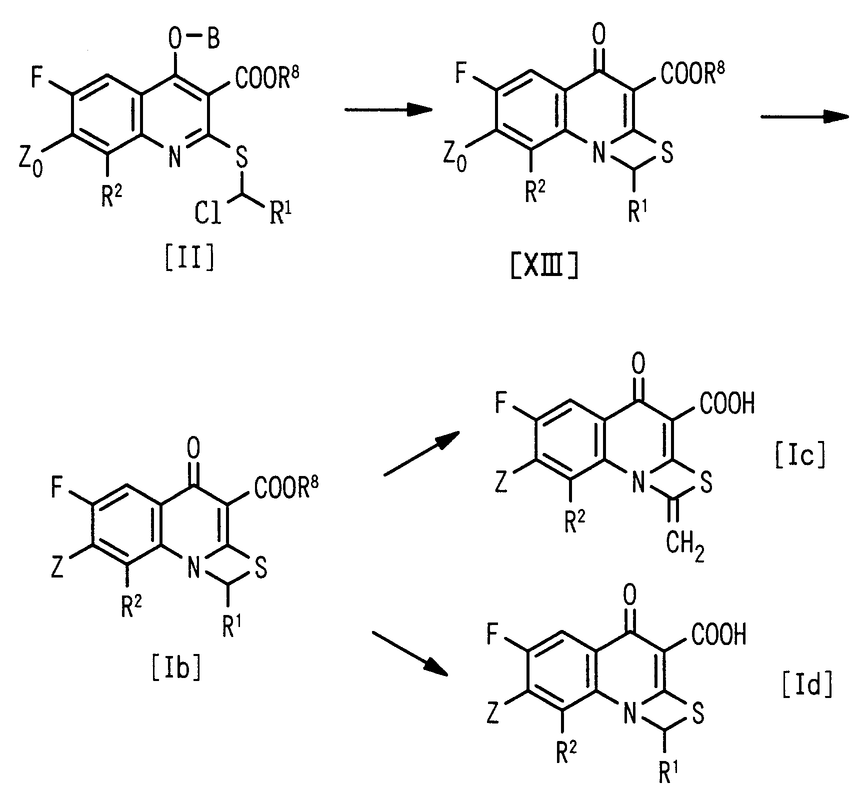
In the above formulas, R¹, R², Z₀ and Z are as defined hereinbefore; R⁸ represents alkyl; B represents a hydroxy-protecting group.

The alkyl represented by R⁸ is preferably a straight-chain or branched-chain alkyl group of 1-10 carbon atoms, including but not limited to methyl, ethyl, n-propyl, isopropyl, n-butyl, isobutyl, sec-butyl, tert-butyl, n-amyl and n-octyl.

The halogen Z₀ is preferably fluorine or chlorine.

The hydroxy-protecting group is preferably acyl such as acetyl or benzoyl.

[XIII] can be produced by subjecting [II] to cyclization reaction in the presence of a base generally at ambient temperature to 100°C. In this step, the protective group is simultaneously eliminated. The solvent may be any solvent that does not interfere with the reaction. Thus, for example, hydrocarbons such as benzene and toluene, ethers such as tetrahydrofuran and dioxane, aprotic solvents such as N,N-dimethylformamide, N,N-dimethylacetamide, dimethyl sulfoxide and sulfolane as well as mixtures thereof can be used. The base may be an organic base (e.g. sodium acetate, triethylamine, tripropylamine, pyridine, etc.) or an inorganic base (e.g. lithium hydroxide, sodium hydrogen carbonate, sodium hydroxide, succinimide sodium salt, α-pyrrolidone sodium salt, etc.). The base is generally used in a proportion of 1-10 equivalents relative to [II]. The reaction time is dependent on the species of [II], base and solvent but generally ranges from 30 minutes to 180 hours.

The compound of the present invention having a fluoroalkyl group in the 1-position can be produced by the following process as well. 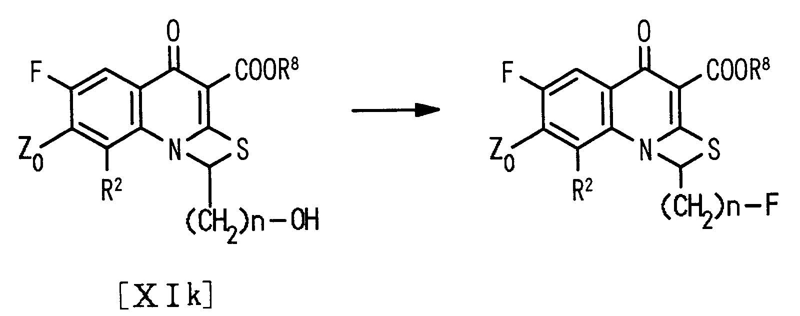
[R², Z₀, R⁸ and n are as defined hereinbefore]
Thus, the desirable compound can be produced by permitting to react [XIk] with a fluorinating agent in a solvent generally at -78°C to 250°C, preferably 0°C to 170°C for 30 minutes - 500 hours, preferably 1 - 120 hours. The solvent that can be used includes aprotic solvents such as N,N-dimethylformamide, dimethyl sulfoxide, sulfolane, methylene chloride, chloroform and tetrahydrofuran. The fluorinating agent that can be used includes sulfur fluorides such as diethylaminosulfur trifluoride (DAST), morpholylsulfur trifluoride, sulfur tetrafluoride, sulfuryl fluoride, sulfuryl chloride fluoride and thionyl fluoride, phosphorus fluorides such as phenyltetrafluorophosphorane, diphenyltrifluorophosphorane, difluorotriphenylphosphorane, fluorotetrabutylsulfolane, tetrabutylphosphonium hydrogen bisulfide and triphenylphosphonium trifluoride, ammonium fluoride compound such as tetrabutylammonium fluoride, alkali metal compounds such as potassium fluoride, potassium hydrofluoride, cesium fluoride, sodium fluoride and sodium hydrofluoride. The amount of the fluorinating agent is 1-10 equivalents, preferably 1-5 equivalents. To control the fluorination reaction, a hydrohalic acid such as hydrofluoric acid, hydrochloric acid or hydrobromic acid, preferably hydrofluoric acid, can be employed.

In lieu of [XIk], a sulfuric acid ester available on substitution of the hydroxyl group with methanesulfonyl, p-toluenesulfonyl, trifluoromethanesulfonyl or 1-imidazolylsulfonyl can likewise be employed. In this case, the solvent that can be used includes alcohols such as methanol, ethanol, isopropyl alcohol and butanol, in addition to the solvents mentioned hereinbefore.

The compound [Ib] having a cyclic amino group at the 7-position [Z : cyclic amino] can also be produced by subjecting the compound [II] having a halogen atom at the 7-position [Z₀ : halogen] to a similar reaction to give a 6-fluoro-7-halothiazetoquinolinecarboxylic acid derivative [XIII] and, then, permitting to react this derivative with a cyclic amine in the presence of a base. This reaction with a cyclic amine can be carried out by, for example, permitting said 6-fluoro-7-halo compound [XIII] to react with the cyclic amine in the presence of a base (e.g. potassium carbonate, sodium carbonate, potassium hydrogen carbonate, sodium hydrogen carbonate, pyridine, triethylamine, etc.) in a solvent (e.g. N,N-dimethylformamide, dimethyl sulfoxide, acetonitrile, pyridine, etc.) or using an excess of the amine in lieu of said base. The reaction temperature is generally -20°C to 100°C. Relative to 6-fluoro-7-halo compound [XIII], the amine is used in a proportion of 1-5 equivalents and the base is used in a proportion of 1-10 equivalents.

The 8-substituted derivative can also be produced by introducing a desired substituent group, by the per se known procedure, into the 8-position of the compound [Ib] (R² : hydrogen) obtained in the above manner.

It is apparent that both the compounds [Ib] and [Id] of the present invention have an asymmetric carbon in the respective structures. The optical isomers of these compounds can each be obtained by optical resolution of the racemic compound by means of a chiral column or by using optically active intermediates.

For optical resolution with a chiral column, whichever of a reversed phase column (e.g. Sumichiral OA) or a normal phase column (e.g. Chiralcel OD) can be employed. The eluent may for example be an alcohol (e.g. methanol or ethanol), acetonitrile or water and the like when a reversed phase column is used. n-Hexane, ethanol or isopropyl alcohol and the like can be used when a normal phase column is used.

The compound [Ib](R⁸ : alkyl) produced in the above manner can be treated with alkali, if desired, to provide a compound [Ic] having a methylene group at the 1-position (A : >C=CH₂) and a carboxyl group at the 3-position (R³ : hydrogen). This reaction can be conducted by stirring the starting compound in 2-30 volumes (preferably 5-10 volumes) of a 1-5% solution of potassium hydroxide or sodium hydroxide in water or a mixture of water and alcohol (methanol, ethanol, propanol, or butanol, preferably tert-butanol) at ambient temperature to 100°C, preferably the boiling temperature of the solvent used. Furthermore, by acid hydrolysis, said compound can be converted to the compound [Id] having fluoroalkyl at the 1-position (A : >CHR¹) and a carboxyl group at the 3-position (R³ : hydrogen). This hydrolysis reaction is conducted using a large excess of acid (e.g. fuming sulfuric acid, sulfuric acid, hydrochloric acid, hydrobromic acid, hydrobromic acid/acetic acid, chlorosulfonic acid, polyphosphoric acid, etc.), preferably 10-20 volumes of the acid, as the solvent at ambient temperature to 110°C.

The ester [Ib] can be converted to a different ester by stirring it in 10-100 volumes of an alcohol corresponding to the desired ester in the presence of a catalytic amount of concentrated sulfuric acid at 0°C to 150°C, preferably at ambient temperature to 110°C.

In the case of a carboxylic acid (R³ : hydrogen), if desired, it can be esterified to the desired ester (R³ : alkyl). This esterification can be carried out by the per se known esterification reaction, for example using thionyl chloride and an alcohol, an alcohol and a condensing agent (e.g. dicyclohexylcarbodiimide), or an alkyl halide or substituted alkyl halide and an alkoxide. Furthermore, the carboxylic acid can be converted to a pharmacologically acceptable salt (e.g. sodium salt, potassium salt, etc.) by the per se known procedure.

Where the compound produced by the method described above has a 1-piperazinyl group at the 7-position, it can be converted to a 4-substituted piperazinyl compound by introducing the substituent group to the nitrogen atom by the per se known procedure. Typically, it can be converted to a 4-alkyl-1-piperazinyl compound or a 4-(5-alkyl-2-oxo-1,3-dioxol-4-yl)methyl-1-piperazinyl compound by permitting to react it with an alkylating agent or a 5-alkyl-4-halomethyl-1,3-dioxol-2-one in the presence of a base in an anhydrous solvent. The solvent that can be used for this purpose includes but is not limited to N,N-dimethylformamide, dimethyl sulfoxide, tetrahydrofuran, and dioxane. The base may for example be sodium carbonate, potassium carbonate, sodium hydrogen carbonate, potassium hydrogen carbonate, sodium hydroxide, sodium methoxide, sodium ethoxide or sodium amide. This reaction is generally carried out at 0°C-40°C. The proportion of the alkylating agent is generally 1-5 equivalents based on the compound having a 1-piperazinyl group at the 7-position.

The desirable compound thus produced can be isolated and purified by per se known procedures such as concentration, pH adjustment, phase transfer or redistribution, solvent extraction, crystallization, recrystallization, fractional distillation and chromatography, among other techniques.

The starting compound [II] can be produced according to the following reaction schema as will be described in detail in the reference examples. 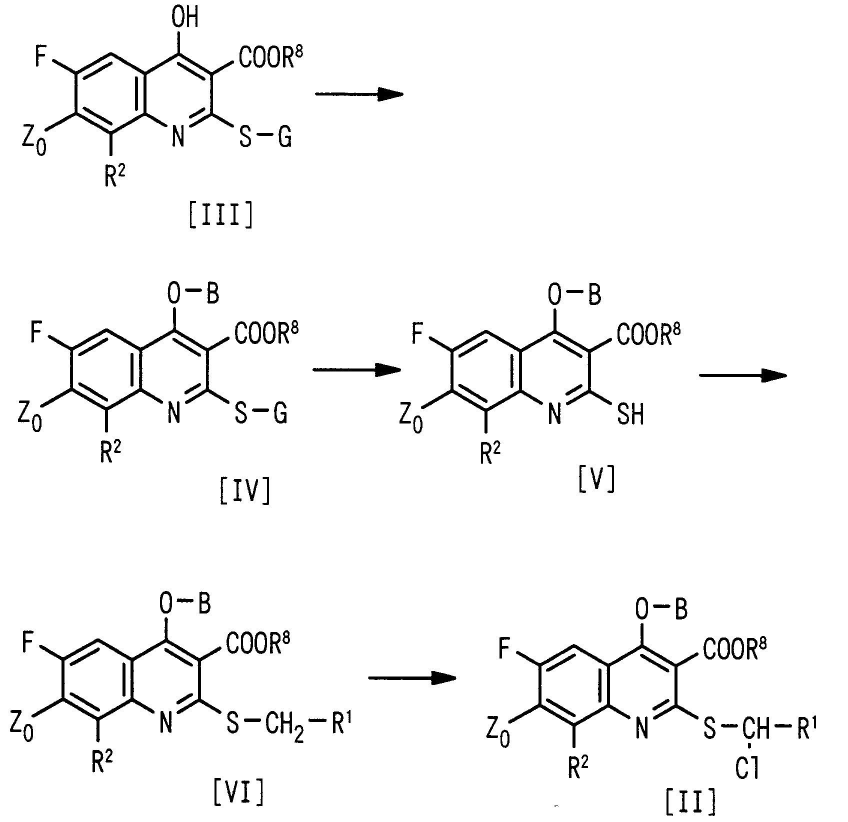
(wherein Z₀, R¹, R², R⁸ and B are as defined hereinbefore; G represents an S-protecting group.)

The sulfur-protecting group that can be used includes but is not limited to alkoxymethyl, substituted benzyl, 2,4-dinitrophenyl, the disulfide which is the dimer of [II], alkylthiomethyl, substituted carbamoyl, diphenylmethyl, triphenylmethyl, picolyl, acetamidomethyl, β,β,β-trifluoro-α-acylaminoethyl, β,β-diethoxycarbonylethyl, acyl (e.g. acetyl, benzoyl), benzoyloxycarbonylethyl, tetrahydropyranyl, benzylthiomethyl and isobutyloxymethyl. Specific representative examples of the above protective group are methoxymethyl and methoxybenzyl.

### Step 1

The 4-hydroxyl group of compound [III] is acylated by the conventional method to provide compound [IV]. This acylation reaction can be conducted at 0-40°C using an acylating agent such as an acyl chloride (e.g. acetyl chloride, benzoyl chloride, etc.) or acetic anhydride-pyridine. The reaction time which is dependent on the species of acylating agent and the reaction temperature is generally 10 minutes to 12 hours.

### Step 2

The 2-protective group of compound [IV] is eliminated by the conventional method to provide compound [V]. Where G is methoxymethyl, for instance, the protective group can be removed by treating [IV] with hydrochloric acid, concentrated sulfuric acid/ethanol, methanesulfonic acid, trifluoromethanesulfonic acid or trifluoroacetic acid, or a mixture thereof, under cooling or at ambient temperature.

### Step 3

Compound [V] is fluoroalkylated to compound [VI]. This fluoroalkylation can be accomplished by permitting to react compound [V] with a halide (e.g. 1-bromo-2-fluoroethane, 1-bromo-3-fluoropropane, 1-bromo-2,2,2-trifluoroethane, etc.) in a reaction-inert solvent in the presence of a base (e.g. sodium carbonate, potassium carbonate, sodium hydrogen carbonate, triethylamine, etc.) generally at 0-120°C. The solvent is preferably an aprotic solvent such as N,N-dimethylformamide (DMF), N,N-dimethylacetamide, dimethyl sulfoxide (DMSO) or sulfolane. The proportions of the dihalide and the base are not less than equimolar, preferably 1.0-2.5 moles, relative to each mol of [V]. To hasten its progress, the reaction may be carried out with addition of 0.01-3.0 moles equivalents of sodium iodide or potassium iodide. Compound [VI] is chlorinated to compound [II].

### Step 4

This chlorination is carried out using a chlorinating agent in a reaction-inert solvent to give [II]. The reaction temperature is generally -30°C to 100°C. The solvent that can be used includes but is not limited to halogenated hydrocarbons such as chloroform, dichloromethane and carbon tetrachloride, hexane, dioxane, and tetrahydrofuran. The chlorinating agent that can be used includes but is not limited to N-chlorosuccinimide, sulfuryl chloride, and molecular chlorine. The chlorinating agent is used generally in an equimolar proportion to excess relative to compound [VI]. The reaction time, which is dependent on the species of compound [VI], chlorinating agent and solvent, the reaction temperature and other conditions, is generally 30 minutes to 3 hours.

The pivotal intermediate [XI] can be produced according to the following reaction schema as will be described hereinafter in the reference examples. 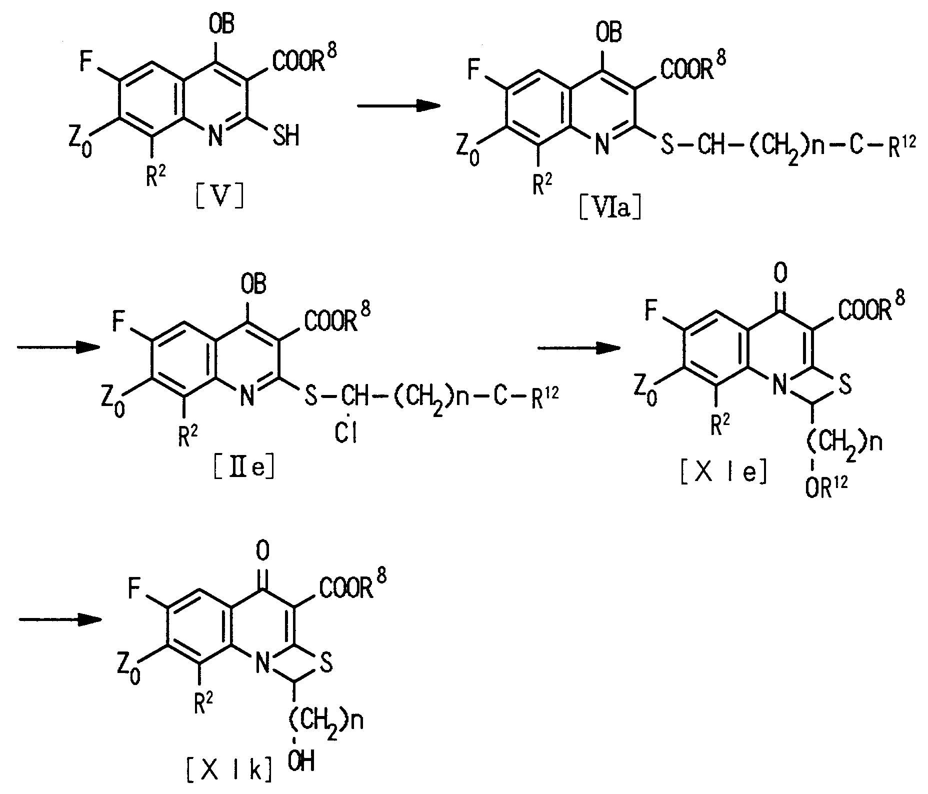
In the formulas, R², R⁸, Z₀, B, R¹² and n are as defined hereinbefore.

The optically active intermediate compounds can be obtained by stereoselective enzymatic esterification at a desired stage as shown in the following reaction schemas. 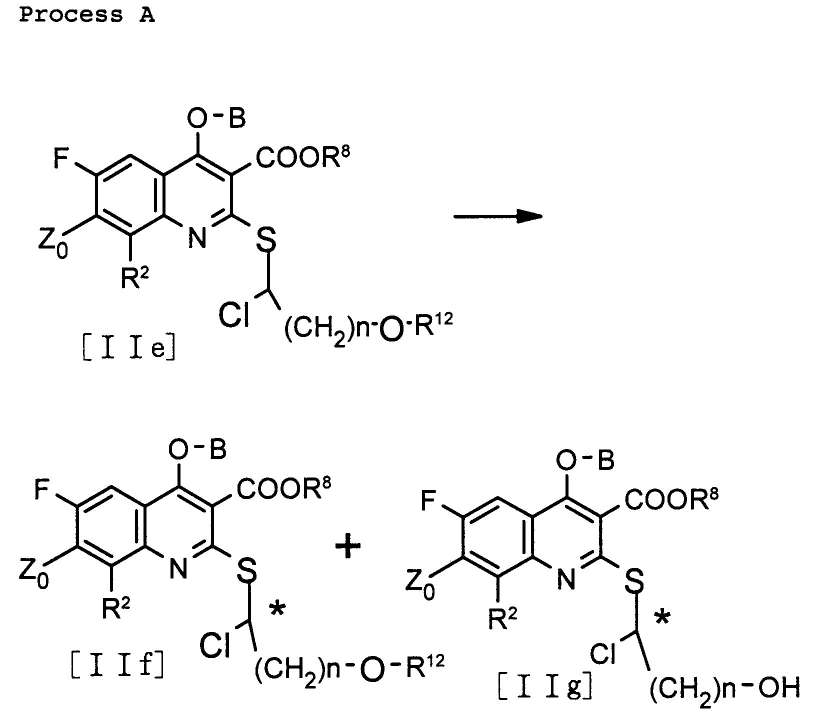
In the formulas, Z₀, R², R⁸, R¹², B and n are as defined hereinbefore. 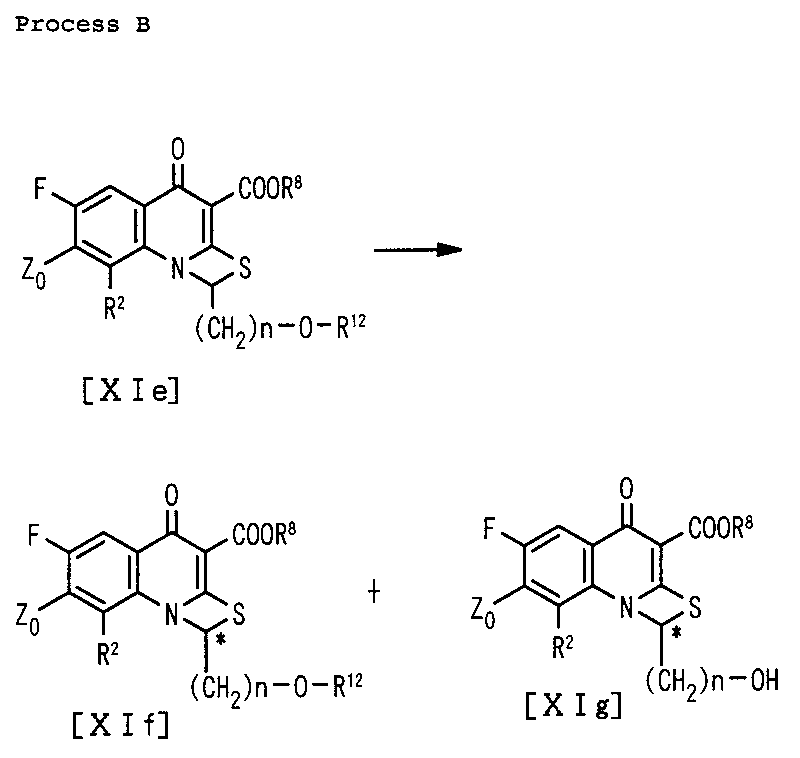
In the formulas, Z₀, R², R⁸, R¹² and n are as defined hereinbefore. 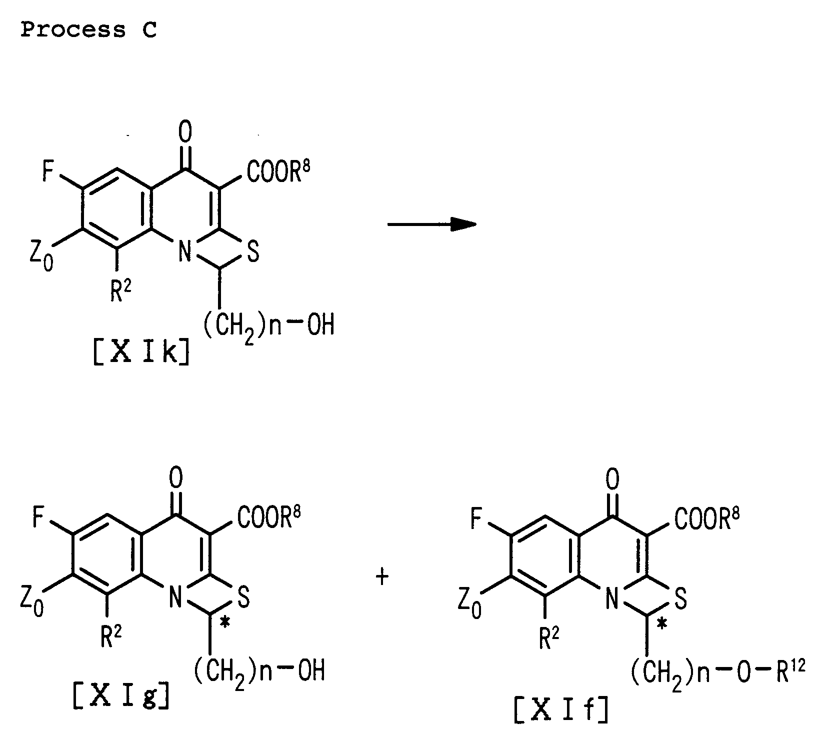
In the formulas, Z₀, R², R⁸, R¹² and n are as defined hereinbefore.

Process A and Process B: An optical isomer of the racemic substrate is subjected to alcoholysis in the presence of an enzyme having stereoselective transferase activity and is separated from the unreacted compound to provide optical isomers of the quinolinethioalkanol derivative and thiazetoquinolinethioalkanol derivative in A and B processes, respectively.

Process C: An optical isomer of the racmic substrate is selectively acylated with an acyl donor compound in the presence of an enzyme having stereoselective transferase activity and the acylated compound is separated from the unreacted compound to provide an optical isomer of the thiazetoquinolinealkanol derivative.

In the biochemical optical resolution of the racemic compound by the enzymatic technique, the asymmetric alcoholysis reaction of the ester in an organic solvent and the asymmetric esterification reaction of the alcohol in an organic solvent can be accomplished. In the presence of an enzyme having stereoselective transesterase activity,An optical isomer of the racemic substrate is selectively permitting to react with an alcohol in an organic solvent and the reaction product is separated from the unreacted compound to provide optical isomers. The alcohol that can be used includes straight-chain or branched-chain aliphatic alcohols or aromatic-substituted alcohols, for example methanol, ethanol, propanol, butanol, heptanol, octanol, benzyl alcohol and so on.

Moreover, in the presence of an enzyme having stereoselective transesterase activity, an optical isomers in the racemic substrate is selectively acylated with an acyl donor in an organic solvent and the acylation product is separated from the unreacted compound to provide optically active compounds. The acyl donor that can be used includes but is not limited to alkenyl esters of aliphatic acids such as vinyl acetate, vinyl butyrate, isopropenyl acetate and isopropenyl butyrate, phenyl esters of aliphatic acids such as phenyl acetate and phenyl butyrate, benzyl esters of aliphatic acids such as benzyl acetate and benzyl butyrate, anhydrides of aliphatic carboxylic, aromatic or cyclic carboxylic acids such as acetic anhydride, propionic anhydride, benzoic anhydride and succinic anhydride, and oxime esters such as acetoxime esters.

As the enzyme exhibiting stereoselective transesterase activity in an organic solvent, for instance, lipase can be employed. The enzyme for use in the present invention may be any enzyme that can react stereospecifically on an optical isomer of the racemic substrate. To be specific, lipases derived from microorganisms of the genera Pseudomonas, Candida, Alcaligenes, Achromobacter, Mucor, etc. can be used. These lipases may be powdery or those immobilized on a carrier such as celite or various ion exchange resins. As commercial products, Lipase OF, QL, QLC, QLG, PL, PLC, PLG, AL, ALC and ALG (manufactured by Meito Sangyo), Lipozyme IM, Novozyme SP (Novo-Nordisk Bio Industries), and Toyozyme LIP (Toyobo) can be used by way of example.

The proportion of the alcohol, enol ester or the like is generally 0.5-200 moles, preferably 1-100 moles, per mol of the racemic compound and the proportion of the enzyme is generally 1-50 g, preferably 2-30 g, per gram of the racemic compound. This reaction is generally conducted under shaking or stirring generally at 5-90°C, preferably 15-65°C, for 1-100 hours, preferably 2-70 hours.

The solvent that can be used includes n-hexane, toluene, diisopropyl ether, tetrahydrofuran, acetonitrile, dioxane and acetone, among other solvents. After the reaction, the enzyme can be recovered by filtration for reuse. The optically active compounds produced by the above reaction can be easily isolated and purified by extraction or column chromatography.

Among the species of the compound of the present invention, the compound which are especially meritorious as therapeutic agents for infectious diseases are the compounds of Examples 12, 17, 18, 22, 23, 29, 33, 36, 59 and 60. The compounds of Examples 12, 17, 18, 29 and 60 are particularly excellent and, among them, the compounds of Examples 29 and 60 are most desirable.

For use of the compound of the present invention as a medicine, it can be administered to animals inclusive of man, either as it is or in the form of a pharmaceutical composition formulated with a medicinally acceptable non-toxic, inert carrier and containing the compound in a concentration of 0.1%-99.5%, preferably 0.5%-90%.

As the carrier, one or more kinds of solid, semi-solid or liquid diluents, fillers and other formulation auxiliary agents can be employed. The pharmaceutical composition is preferably administered in a unit dosage form. The pharmaceutical composition of the present invention can be administered orally, into the tissue, topically (e.g. transdermally) or rectally. The dosage form should of course be compatible with the contemplated route of administration. Oral administration, for instance, is particularly preferred.

The dosage as a therapeutic drug for infectious diseases is preferably adjusted in consideration of patient state such as age and body weight, route of administration, nature and severity of illness, etc. Generally speaking, in terms of the amount of the active ingredient, the daily dosage for an adult human is 50-1000 mg, preferably 100 mg-300 mg. A dosage less than the above may prove sufficient in some cases, while a higher dosage may be required in other cases. The daily dosage is preferably administered in 2-3 divided doses.

### BEST MODE OF PRACTICING THE INVENTION

The following reference examples concerning the production of the compound of the invention, examples concerning the intermediates forming part of the invention (all shown as production examples) and examples and test examples of the final products are intended to describe the present invention in further detail.

### Production Example 1

### Ethyl 6,7-difluoro-1-fluoromethyl-4-oxo-4H-[1,3]thiazeto[3,2-a]quinoline-3-carboxylate

(1) Ethyl 4-benzoyloxy-6,7-difluoro-2-methoxymethyl-thioquinoline-3-carboxylate
In 500 ml of pyridine was dissolved 100 g of ethyl 6,7-difluoro-4-hydroxy-2-methoxymethylthioquinoline-3-carboxylate. Then, 46.95 g of benzoyl chloride was added dropwise with ice-cooling and stirring and the reaction was conducted at the same temperature for 2 hours. To this reaction mixture was added 1.5 l of iced water and the mixture was stirred for a while. The crystals that separated out were collected by filtration, washd with water and dissolved in chloroform. The solution was washed with water, diluted hydrochloric acid, and saturated sodium chloride-water, and concentrated. The procedure provided 132 g of the desirable compound as light-yellow solid. m. p. 98-99°C.
(2) Ethyl 4-benzoyloxy-6,7-difluoro-2-mercaptoquinoline-3-carboxylate
A suspension consisting of 132 g of ethyl 4-benzoyloxy-6,7-difluoro-2-methoxymethylthioquinoline-3- carboxylate, 38 ml of 35% concentrated hydrochloric acid, 1280 ml of ethanol was stirred at ambient temperature for 22 hours. The resulting crystals were collected by filtration, washed with ether, and dried, whereby 102.6 g of the desirable compound was obtained as yellow solid. m. p. 162°C.
(3) Ethyl 4-benzoyloxy-6,7-difluoro-2-(2-fluoroethyl)thioquinoline-3-carboxylate
While a suspension consisting of 53.8 g of ethyl 4-benzoyloxy-6,7-difluoro-2-mercaptoquinoline-3-carboxylate, 11.61 g of sodium hydrogen carbonate and 500 ml of N,N-dimethylformamide was stirred at ambient temperature, 17.54 g of 1-bromo-2-fluoroethane was added dropwise and the reaction was conducted at the same temperature for 17 hours. The reaction mixture was then concenrated under reduced pressure and the residue was diluted with 300 ml of water and extracted with chloroform. The extract was washed with water, dried over magnesium sulfate, and concentrated. The yellow residue was suspended in about 100 ml of ethanol and the suspension was refluxed for 30 minutes. This was cooled and the resulting crystals were collected by filtration, washed with ethanol and ether, and dried to provide 34.17 g of the desirable compound as light-yellow powder. m. p. 124°C.
(4) Ethyl 4-benzoyloxy-2-(1-chloro-2-fluoroethyl)thio-6,7-difluoroquinoline-3-carboxylate
In 350 ml of n-hexane was dissolved 34.17 g of ethyl 4-benzoyloxy-6,7-difluoro-2-(2-fluoroethyl)thioquinoline-3-carboxylate and while the solution was stirred at 40°C, a solution of sulfuryl chloride in n-hexane (27.29 g/100 ml) was added dropwise over 30 minutes. The mixture was stirred at the same temperature for 19 hours. The reaction mixture was then concentrated under reduced pressure and the residue was dissolved in chloroform. This solution was washed with water, dried over magnesium sulfate, and concentrated. The residue was subjected to column chromatography (Wakogel C-200, chloroform) to provide 22 g of the desirable compound as brown liquid.
(5) Ethyl 6,7-difluoro-1-fluoromethyl-4-oxo-4H-[1,3]thiazeto[3,2-a]quinoline-3-carboxylate
A combined solution of 22 g of ethyl 4-benzoyloxy-2-(1-chloro-2-fluoroethyl)thio-6,7-difluoroquinoline-3-carboxylate, 12.2 g of triethylamine, and 250 ml of tetrahydrofuran was stirred at a bath temperature of 60°C or below for 60 hours. The crystals that separated out were collected by filtration, washed with ether and dried to provide 15.02 g of the desirable compound as light-gray powder. m. p. 225°C

| Elemental analysis for C₁₄H₁₀F₃NO₃S | | | |
|---|---|---|---|
| Calcd. (%): | C, 51.06; | H, 3.06; | N, 4.25 |
| Found (%): | C, 51.09; | H, 3.12; | N, 4.25 |

### Production Example 2

### Ethyl 6,7,8-trifluoro-1-fluoromethyl-4-oxo-4H-[1,3]thiazeto[3,2-a]quinoline-3-carboxylate

Using ethyl 6,7,8-trifluoro-4-hydroxy-2-methoxymethylthioquinoline-3-carboxylate, the procedure of Production Example 1 was otherwise repeated to provide the desirable compound.
m. p. 227°C

### Production Example 3

### Ethyl 6,7-difluoro-1-fluoromethyl-8-methoxy-4-oxo-4H-[1,3]thiazeto[3,2-a]quinoline-3-carboxylate

Using ethyl 6,7-difluoro-4-hydroxy-8-methoxy-2-methoxymethylthioquinoline-3-carboxylate, the procedure of Production Example 1 (1)-(5) was otherwise repeated to provide the desirable compound. m. p. 231-233°C

### Production Example 4

### Ethyl 6,7-difluoro-1-fluoromethyl-4-oxo-4H-[1,3]thiazeto[3,2-a]quinoline-3-carboxylate (optical resolution by HPLC)

Using chiralcel OD (Daicel, 2.0 cmD x 25 cmL) as an optically active column, 805 mg of the racemic compound obtained in Production Example 1 was injected at a rate of 5 mg/5ml (isopropyl alcohol) and using a solvent mixture of n-hexane and 2-propanol (75:25) as the mobile phase, elution was carried out at a flow rate of 7.6 ml/min. at 40°C. 254 nm UV light was used for detection. The compound with a retention time of 58.917 minutes amounted to 364 mg and the compound with a retention time of 74.214 minutes amounted to 377 mg.
(+)-compound: m. p. 221-225°C
Specific rotation [α] _{D} = +94.52° (DMF, c = 0.493)

| Elemental analysis for C₁₄H₁₀F₃NO₃S | | | |
|---|---|---|---|
| Calcd. (%): | C, 51.06; | H, 3.06; | N, 4.25 |
| Found (%): | C, 51.86; | H, 3.42; | N, 4.38 |

(-)-compound: m. p. 209-211°C
Specific rotation [α] _{D} = -105.82° (DMF, c = 0.429)

| Elemental analysis for C₁₄H₁₀F₃NO₃S | | | |
|---|---|---|---|
| Calcd. (%): | C, 51.06; | H, 3.06; | N, 4.25 |
| Found (%): | C, 52.84; | H, 3.22; | N, 4.30 |

### Production Example 5

### n-Butyl 6,7-difluoro-1-fluoromethyl-4-oxo-4H-[1,3]thiazeto[3,2-a]quinoline-3-carboxylate (optical resolution by HPLC)

The desirable compound was obtained using the racemic compound and the chiral column as in Production Example 4. (+)-compound: m. p. 185°C
Specific rotation [α] _{D} = +98.92° (DMF, c = 0.558)

| Elemental analysis for C₁₆H₁₄F₃NO₃S | | | |
|---|---|---|---|
| Calcd. (%): | C, 53.78; | H, 3.95; | N, 3.92 |
| Found (%): | C, 53.84; | H, 4.20; | N, 3.90 |

(-)-compound: m. p. 186°C
Specific rotation [α] _{D} = -104.69° (DMF, c = 0.533)

| Elemental analysis for C₁₆H₁₄F₃NO₃S | | | |
|---|---|---|---|
| Calcd. (%): | C, 53.78; | H, 3.95; | N, 3.92 |
| Found (%): | C, 54.02; | H, 3.73; | N, 3.87 |

### Production Example 6

### Ethyl 6,7-difluoro-8-methoxy-1-methylene-4-oxo-4H-[1,3]thiazeto[3,2-a]quinoline-3-carboxylate

A suspension of 3.00 g of the ethyl 6,7-difluoro-1-fluoromethyl-8-methoxy-4-oxo-4H-[1,3]thiazeto[3,2- a]quinoline-3-carboxylate obtained in Production Example 3 in 60 ml of 1% potassium hydroxide/n-butanol-water (1:1) was stirred at 60°C for 15 hours. The reaction mixture was then concentrated under reduced pressure and the residue was suspended in 100 ml of water and extracted 3 times with chloroform-methanol (20:1). The organic layer was washed with saturated aqueous sodium chloride solution, dried over magnesium sulfate, and concentrated. The residue was purified by column chromatography (C-200, CHCl₃ : MeOH = 200:1 → 100:1) to provide 0.61 g of the desirable compound. m. p. 217-218 °C

| Elemental analysis for C₁₅H₁₁F₂NO₄S | | | |
|---|---|---|---|
| Calcd. (%): | C, 53.10; | H, 3.27; | N, 4.13 |
| Found (%): | C, 53.10; | H, 3.27; | N, 4.02 |

### Production Example 7

### 6,7-Difluoro-8-methoxy-1-methylene-4-oxo-4H-[1,3]thiazeto[3,2-a]quinoline-3-carboxylic acid

The aqueous layer separated in Production Example 6 was acidified (pH 3-4) with 10 % hydrochloric acid under ice-cooling and stirring and extracted 3 times with chloroform-methanol (20:1). The extract was washed with saturated aqueous sodium chloride solution, dried over magnesium sulfate, and concentrated to provide 1.57 g of the desirable compound. m. p. 237-238°C

| Elemental analysis for C₁₃H₁₇F₂NO₄S | | | |
|---|---|---|---|
| Calcd. (%): | C, 50.16; | H, 2.27; | N, 4.50 |
| Found (%): | C, 50.36; | H, 2.12; | N, 4.49 |

### Production Example 8

### Synthesis of ethyl 1-acetoxymethyl-6,7-difluoro-4-oxo-4H-[1,3]thiazeto[3,2-a]quinoline-3-carboxylate

(1) Synthesis of ethyl 2-(2-acetoxyethyl)thio-4-benzoyloxy-6,7-difluoroquinoline-3-carboxylate
In 1.5 l of DMF were suspended 194.69 g of ethyl 4-benzoyloxy-6,7-difluoro-2-mercaptoquinoline-3-carboxylate and 42.005 g of sodium hydrogen carbonate. Then, with stirring at ambient temperature, 83.505 g of 1-acetoxy-2-bromoethane was added over 45 minutes and the reaction was conducted for 30 minutes. The solvent was distilled off from the reaction mixture and the residue was diluted with water and extracted with chloroform. The extract was washed with water, dried over magnesium sulfate, and concentrated. The residue was washed with n-hexane and recrystallized from isopropyl alcohol to provide 120.6 g of crystals. m. p. 97°C
(2) Synthesis of ethyl 2-(2-acetoxy-1-chloroethyl)thio-4-benzoyloxy-6,7-difluoroquinoline-3-carboxylate
To a mixture of 30.0 g of the compound obtained in (1) and 300 ml of methylene chloride under ice-cooling and stirring was added sulfuryl chloride dropwise over 30 minutes. After the dropwise addition, the mixture was stirred at ambient temperature for 4 hours. Then, the solvent was distilled off and the crystalline residue was washed with n-hexane and recrystallized from isopropyl ether to provide 19.0 g of crystals.
m. p. 106-107°C
(3) Synthesis of ethyl 1-acetoxymethyl-6,7-difluoro-4-oxo-4H-[1,3]thiazeto[3,2-a]quinoline-3-carboxylate
Amixture of 16.80 g of the compound obtained in (2), 10.00 g of triethylamine, 0.594 g of water, and 200 ml of tetrahydrofuran was refluxed at 80°C overnight. This reaction mixture was cooled and the resulting crystals were collected by filtration, washd with water, and dried to provide 9.6 g of the desirable compound. m. p. 230-231°C

| Elemental analysis for C₁₆H₁₃F₂NO₅S | | | |
|---|---|---|---|
| Calcd. (%): | C, 52.03; | H, 3.55; | N, 3.79 |
| Found (%): | C, 51.92; | H, 3.69; | N, 3.77 |

### Production Example 9

### Ethyl 1-benzoyloxymethyl-6,7-difluoroquinoline-4-oxo-4H-[1,3]thiazeto[3,2-a]quinoline-3-carboxylate

Using 2-bromo-1-benzoyloxyethane, the procedure of Production Example 8 (1)-(3) was otherwise repeated to provide the desirable compound. m. p. 218-219°C

| Elemental analysis for C₂₁H₁₅F₂NO₅S | | | |
|---|---|---|---|
| Calcd. (%): | C, 58.47; | H, 3.50; | N, 3.25 |
| Found (%): | C, 58.63; | H, 3.38; | N, 3.25 |

### Production Example 10

### Synthesis of ethyl 1-acetoxymethyl-6-fluoro-7-(4-methyl-1-piperazinyl)-4-oxo-4H-[1,3]thiazeto[3,2-a]quinoline-3-carboxylate

In 20 ml of DMF were suspended 1.004 g of the compound obtained in Production Example 8 (3) and 1.356 g of N-methylpiperazine and the suspension was stirred at ambient temperature for 32 hours. The reaction mixture was then concentrated under reduced pressure and the residue was extracted with chloroform. The extract was washed with saturated aqueous sodium chloride solution, dried, and concentrated under reduced pressure. The residue was purified by column chromatography (silica gel C-200, eluent=chloroform → methanol : chloroform (2:98)) to provide 340 mg of the desirable compound as colorless powder.
m. p. 223°C

| Elemental analysis for C₂₁H₂₄FN₃O₅S | | | |
|---|---|---|---|
| Calcd. (%): | C, 56.11; | H, 5.38; | N, 9.35 |
| Found (%): | C, 55.82; | H, 5.37; | N, 9.05 |

### Production Example 11

### Ethyl 1-benzoyloxymethyl-6-fluoro-7-(4-methyl-1-piperazinyl)-4-oxo-4H-[1,3]thiazeto[3,2-a]quinoline-3-carboxylate

A colorless powder was obtained using the compound of Production Example 9 and N-methylpiperazine in otherwise the same manner as Production Example 10.
m. p. 215-216°C

| Elemental analysis for C₂₆H₂₆FN₃O₅S | | | |
|---|---|---|---|
| Calcd. (%): | C, 61.04; | H, 5.12; | N, 8.21 |
| Found (%): | C, 60.98; | H, 5.28; | N, 8.29 |

### Production Example 12

### Synthesis of ethyl 6,7-difluoro-1-hydroxymethyl-4-oxo-4H-[1,3]thiazeto[3,2-a]quinoline-3-carboxylate

A suspension of 12.25 g of the compound obtained in Production Example 8 (3) in 250 ml of 1% H₂SO₄/EtOH was stirred at 80°C for 5 hours. This reaction mixture was then centrifuged and separated into the supernatant and crystals. The supernatant was removed by decantation. The residual crystals were washed with a mixture of ethanol and water and subjected to centrifugation and decantation 3 times. Finally, the crystals were washed with ethanol and water, filtered through a glass filter, and dried in vacuo to provide 9.35 g of white powder. m. p. 288-290°C (decomp.)

| Elemental analysis for C₁₄H₁₁F₂NO₄S | | | |
|---|---|---|---|
| Calcd. (%): | C, 51.37; | H, 3.39; | N, 4.28 |
| Found (%): | C, 51.57; | H, 3.14; | N, 4.31 |

### Production Example 13

### Methyl 6,7-difluoro-1-hydroxymethyl-4-oxo-4H-[1,3]thiazeto[3,2-a]quinoline-3-carboxylate (transesterification)

In 15 ml of methanol was suspended 554 mg of ethyl 1-acetoxymethyl-6,7-difluoro-4-oxo-4H-[1,3]thiazeto[3,2-a]quinoline-3-carboxylate and the suspension was refluxed for 17 hours. After cooling, the crystals separated out were collected by filtration. This crystal crop was washed 3 times with water, 3 times with methanol, and 3 times with ether and dried under reduced pressure at 60-70°C for 7 hours. This procedure provided 420 mg of the desirable compound as colorless powder.
m. p. 294-295°C (decomp.)

| Elemental analysis for C₁₃H₁₉F₂NO₄S | | | |
|---|---|---|---|
| Calcd. (%): | C, 49.84; | H, 2.90; | N, 4.47 |
| Found (%): | C, 49.70; | H, 2.63; | N, 4.42 |

The following compounds were synthesized in the same way as above using the corresponding alcohols.

### Production Example 14

### Isopropyl 6,7-difluoro-1-hydroxymethyl-4-oxo-4H-[1,3]thiazeto[3,2-a]quinoline-3-carboxylate

m. p. 274-275°C

| Elemental analysis for C₁₅H₁₃F₂NO₄S | | | |
|---|---|---|---|
| Calcd. (%): | C, 52.78; | H, 3.84; | N, 4.10 |
| Found (%): | C, 52.99; | H, 3.53; | N, 4.02 |

### Production Example 15

### n-Butyl 6,7-difluoro-1-hydroxymethyl-4-oxo-4H-[1,3]thiazeto[3,2-a]quinoline-3-carboxylate

m. p. 268-271°C

| Elemental analysis for C₁₆H₁₅F₂NO₄S | | | |
|---|---|---|---|
| Calcd. (%): | C, 54.08; | H, 4.25; | N, 3.94 |
| Found (%): | C, 53.78; | H, 4.37; | N, 3.84 |

### Production Example 16

### n-Amyl 6,7-difluoro-1-hydroxymethyl-4-oxo-4H-[1,3]thiazeto[3,2-a]quinoline-3-carboxylate

m. p. 237-239°C

| Elemental analysis for C₁₇H₁₇F₂NO₄S | | | |
|---|---|---|---|
| Calcd. (%): | C, 55.28; | H, 4.64; | N, 3.79 |
| Found (%): | C, 55.63; | H, 4.64; | N, 3.70 |

### Production Example 17

### n-Octyl 6,7-difluoro-1-hydroxymethyl-4-oxo-4H-[1,3]thiazeto[3,2-a]quinoline-3-carboxylate

m. p. 236-238°C

| Elemental analysis for C₂₀H₂₃F₂NO₄S | | | |
|---|---|---|---|
| Calcd. (%): | C, 58.36; | H, 5.63; | N, 3.40 |
| Found (%): | C, 58.33; | H, 5.72; | N, 3.44 |

### Production Example 18

### Synthesis of ethyl 6-fluoro-1-hydroxymethyl-7-(4-methyl-1-piperazinyl)-4-oxo-4H-[1,3]thiazeto[3,2-a]quinoline-3-carboxylate

In 20 ml of DMF were suspended 1.004 g of the compound obtained in Production Example 12 and 0.9436 g of N-methylpiperazine and the suspension was stirred at 55°C for 60 hours. The reaction mixture was then concentrated under reduced pressure and the residue was extracted with chloroform-methanol (80:20). The organic layer was washed with saturated aqueous sodium chloride solution, dried, and concentrated under reduced pressure. The solid residue was recrystallized from acetonitrile to provide 724 mg of the desirable compound. m. p. 263-265°C

| Elemental analysis for C₁₉H₂₂FN₃O₄S | | | |
|---|---|---|---|
| Calcd. (%): | C, 56.01; | H, 5.44; | N, 10.31 |
| Found (%): | C, 55.97; | H, 5.25; | N, 10.14 |

The following compound was obtained by the same procedure.

### Production Example 19

### Ethyl 6-fluoro-1-hydroxymethyl-7-(1-piperazinyl)-4-oxo-4H-[1,3]thiazeto[3,2-a]quinoline-3-carboxylate

Light-yellow powder, m. p. 217-218°C (decomp.)

| Elemental analysis for C₁₈H₂₀FN₃O₄S·1/2H₂O | | | |
|---|---|---|---|
| Calcd. (%): | C, 53.72; | H, 5.26; | N, 10.44 |
| Found (%): | C, 53.39; | H, 4.98; | N, 10.24 |

### Production Example 20

### Ethyl (3S)-(-)-6-fluoro-1-hydroxymethyl-7-(3-amino-1-pyrrolidinyl)-4-oxo-4H-[1,3]thiazeto[3,2-a]quinoline-3-carboxylate

Using (3S)-(-)-3-amino-1-pyrrolidine, the procedure of Production Example 18 was otherwise repeated to provide the desirable compound. Light-brown powder, m. p. 165-167°C (decomp.)

| Elemental analysis for C₁₈H₂₀FN₃O₄S·H₂O | | | |
|---|---|---|---|
| Calcd. (%): | C, 52.54; | H, 5.39; | N, 10.21 |
| Found (%): | C, 52.83; | H, 5.23; | N, 10.56 |

### Production Example 21

### 6-Fluoro-1-hydroxymethyl-7-(4-methyl-1-piperazinyl)-4-oxo-4H-[1,3]thiazeto[3,2-a]quinoline-3-carboxylic acid hydrochloride

In 8.3 ml of 1% aueous potassium hydroxide solution was suspended 200.4 mg of the compound obtained in Production Example 18 and the suspension was stirred at 55°C for 16 hours. This reaction mixture was filtered and the filtrate was neutralized with 1N-hydrochloric acid. The resulting precipitate was collected, washd with water, and dried to provide 107 mg of the desirable compound as colorless powder. m. p. 252-253°C (decomp.)

| Elemental analysis for C₁₇H₁₈FN₃O₄S·HCl | | | |
|---|---|---|---|
| Calcd. (%): | C, 49.10; | H, 4.60; | N, 10.10 |
| Found (%): | C, 49.06; | H, 4.36; | N, 9.83 |

The following compounds were obtained in the same manner as above.

### Production Example 22

### 6-Fluoro-1-hydroxymethyl-7-(1-piperazinyl)-4-oxo-4H-[1,3]thiazeto[3,2-a]quinoline-3-carboxilic acid Light-yellow powder, m. p. 180°C (decomp.)

| Elemental analysis for C₁₆H₁₆FN₃O₄S·5/4H₂O | | | |
|---|---|---|---|
| Calcd. (%): | C, 49.54; | H, 4.80; | N, 10.83 |
| Found (%): | C, 49.63; | H, 5.09; | N, 10.72 |

### Production Example 23

### (3S)-(-)-6-Fluoro-1-hydroxymethyl-7-(3-amino-1-pyrrolidinyl)-4-oxo-4H-[1,3]thiazeto[3,2-a]quinoline-3-carboxylic acid

Colorless solid m. p. ³300°C

| Elemental analysis for C₁₆H₁₆FN₃O₄S·H₂O | | | |
|---|---|---|---|
| Calcd. (%): | C, 50.01; | H, 4.73; | N, 10.96 |
| Found (%): | C, 49.95; | H, 4.82; | N, 10.82 |

IR cm⁻¹ 3400, 2950, 1630, 1600, 1570, 1510, 1380, 625

### Production Example 24

### Synthesis of methyl 1-acetoxymethyl-6,7-difluoro-4-oxo-4H-[1,3]thiazeto[3,2-a]quinoline-3-carboxylate

In 10 ml of pyridine was dissolved 300 mg of methyl 1-hydroxymethyl-6,7-difluoro-4-oxo-4H-[1,3]thiazeto[3,2-a]quinoline-3-carboxylate obtained in Production Example 13, followed by addition of 300 mg of acetic anhydride, and the mixture was stirred at ambient temperature overnight. The pyridine was distilled off from the reaction mixture under reduced pressure and the residue was washed serially with n-hexane, water, methanol and ether, and dried under reduced pressure at 70°C for 5 hours. As a result, 330 mg of the desirable compound was obtained as brownish powder.
m. p. 220.5-223.0°C (decomp.)

| Elemental analysis for C₁₅H₁₁F₂NO₅S | | | |
|---|---|---|---|
| Calcd. (%): | C, 50.71; | H, 3.12; | N, 3.94 |
| Found (%): | C, 50.69; | H, 2.86; | N, 3.95 |

The following compound was obtained in the same manner as above.

### Production Example 25

### n-Butyl 1-acetoxymethyl-6,7-difluoro-4-oxo-4H-[1,3]thiazeto[3,2-a]quinoline-3-carboxylate

m. p. 178-179°C

| Elemental analysis for C₁₈H₁₇F₂NO₅S | | | |
|---|---|---|---|
| Calcd. (%): | C, 54.40; | H, 4.31; | N, 3.52 |
| Found (%): | C, 54.63; | H, 4.09; | N, 3.78 |

The following compounds can be obtained in the same manner as above.

Isopropyl 1-acetoxymethyl-6,7-difluoro-4-oxo-4H-[1,3]thiazeto[3,2-a]quinoline-3-carboxylate
n-Amyl 1-acetoxymethyl-6,7-difluoro-4-oxo-4H-[1,3]thiazeto[3,2-a]quinoline-3-carboxylate
n-Octyl 1-acetoxymethyl-6,7-difluoro-4-oxo-4H-[1,3]thiazeto[3,2-a]quinoline-3-carboxylate

### Production Example 26

### Ethyl 6,7-difluoro-1-fluoromethyl-4-oxo-4H-[1,3]thiazeto[3,2-a]quinoline-3-carboxylate

While a suspension of 1.00 g of ethyl 6,7-difluoro-1-hydroxymethyl-4-oxo-4H-[1,3]thiazeto[3,2-a]quinoline-3-carboxylate in N,N-dimethylformamide (DMF) was stirred vigorously under ice-cooling, a DMF solution of 0.54 g of DAST (5 ml) was slowly added. The reaction mixture was gradually allowed to return to ambient temperature and further stirred for 15 hours. The reaction mixture was then concentrated under reduced pressure and the residue was dissolved in chloroform-methanol (20:1), washed with water and saturated aqueous sodium chloride solution, and concentrated. The residue was purified by silica gel column chromatography (C-200: chloroform : methanol = 100:1 → 50:1) to provide 180 mg of the desirable compound.
m. p. 225°C-226°C
In elemental analysis, this product was identical with the compound obtained in Production Example 1 (5).

### Production Example 27

### Ethyl 6,7-difluoro-1-acetoxymethyl-4-oxo-4H-[1,3]thiazeto[3,2-a]quinoline-3-carboxylate (production of optical isomers)

(1a) Ethyl 2-(2-hydroxy-1-chroloethyl)thio-4-benzoyloxy-6,7-difluoroquinoline-3-carboxylate (production of optical isomers - Process A)
In 1 ml of THF was dissolved 1 mg of ethyl 2-(2-acetoxy-1-chloroethyl)thio-4-benzoyloxy-6,7-difluoroquinoline-3-carboxylate, a racemic compound, followed by addition of 20 µl of ethanol. Then, 20 mg of Lipase QL (Meito Sangyo) was added and the asymmetric alcoholysis reaction was conducted at 30°C with stirring for 18 hours.
The resulting alcohol and the unreacted compound were determined using a chiral HPLC column to determine the respective optical purities and the rate of conversion (%). HPLC conditions
- Column:: Daicel Chiralcel OD, 4.6 ⌀ x 250 mm
- Eluent solvent:: hexane : 2-propanol = 9 : 1
- Flow rate:: 0.5 ml/min.
- Detector:: Shimadzu UV detector SPD-6A, wavelength 254 nm
- Optical purity:: acetoxy compound 97.7%ee, alcohol compound 61.3%ee
- Conversion rate:: 61.4%

(1b) In 320 ml of acetonitrile was dissolved 4 g of ethyl 2-(2-acetoxy-1-chloroethyl)thio-4-benzoyloxy-6,7-difluoroquinoline-3-carboxylate, a racemic compound, followed by addition of 80 ml of ethanol. Then, 20 g of Lipase QL (Meito Sangyo) was added and the asymmetic alcoholysis reaction was carried out at 30°C with stirring for 5 hours. The resulting (-)-alcohol and the unchanged (+)-acetoxy compound were determined using a chiral HPLC column and the respective optical purities and the rate of conversion (%) were determined. The HPLC conditions were the same as described hereinbefore.
- Conversion rate:: 54.9%
- Optical purity:: acetoxy compound 99.1%ee(-)
alcohol compound 81.5%ee(+)
Then, this reaction mixture was filtered through a 0.45 µ membrane filter (Millipore) to remove the enzyme and fractionally purified by the following method.
(2) Ethyl 2-(2-acetoxy-1-chloroethyl)thio-4-benzoyloxy-6,7-difluoroquinoline-3-carboxylate (isolation of optical isomers)
The reaction mixture was concentrated under reduced pressure and purified by column chromatography (Wakogel C-200; n-hexane : ethyl acetate = 10:1) to provide 900 mg of (-)-acetoxy compound. The hydroxy compound was decomposed within the column and 810 mg of ethyl 4-benzoyloxy-6,7-difluoro-2-mercaptoquinoline-3-carboxylate was recovered.
Acetoxy compound: m. p. 75°C
Specific rotation [α]_{D} = -99.26° (CHCl₃, c=1.094)

| Elemental analysis for C₂₃H₁₈ClF₂NO₆S | | | |
|---|---|---|---|
| Calcd. (%): | C, 54.18; | H, 3.56; | N, 2.75 |
| Found (%): | C, 54.37; | H, 4.08; | N, 2.56 |

(3) Ethyl 1-acetoxymethyl-6,7-difluoro-4-oxo-4H-[1,3]thiazeto[3,2-a]quinoline-3-carboxylate (production of optical isomers)
Using 700 mg of the (-)-acetoxy compound obtained in (2) above, the procedure of Example 8 (3) was otherwise repeated to provide 300 mg of the desirable compound as colorless powder. m. p. 246°C
Specific rotation [α]_{D} = +158.09° (DMF, c=0.525)

| Elemental analysis for C₁₆H₁₃F₂NO₅S·1/2H₂O | | | |
|---|---|---|---|
| Calcd. (%): | C, 50.79; | H, 3.73; | N, 3.70 |
| Found (%): | C, 51.50; | H, 3.99; | N, 3.74 |

(4) Ethyl (+)-1-hydroxymethyl-6,7-difluoro-4-oxo-4H-[1,3]thiazeto[3,2-a]quinoline-3-carboxylate (production of optical isomer)
The (+)-acetoxy compound obtained in (3) above was hydrolyzed to provide the desirable compound.
m. p. 293-296°C (decomp.)
Specific rotation [α]_{D} = +133.95° (DMSO, c=0.745)

| Elemental analysis for C₁₄H₁₁F₂NO₄S | | | |
|---|---|---|---|
| Calcd. (%): | C, 51.37; | H, 3.39; | N, 4.28 |
| Found (%): | C, 51.68; | H, 3.49; | N, 4.33 |

### Production Example 28a

### Ethyl 6,7-difluoro-1-hydroxymethyl-4-oxo-4H-[1,3]thiazeto[3,2-a]quinoline-3-carboxylate and ethyl 1-acetoxymethyl-6,7-difluoro-4-oxo-4H-[1,3]thiazeto[3,2-a]quinoline-3-carboxylate (production of optical isomers - Process B)

(1a) In 1 ml of THF was dissolved 1 mg of ethyl 1-acetoxymethyl-6,7-difluoro-4-oxo-4H-[1,3]thiazeto[3,2-a]quinoline-3-carboxylate, a racemic compound, followed by addition of 20 µl of ethanol. Then, 20 mg of Lipase QL (Meito Sangyo) was added and the mixture was stirred at 30°C for 3 hours for asymmetric alcoholysis.
The resulting hydroxy compound and the acetoxy compound were determined using a chiral HPLC column and the respective optical purities and the rate of conversion (%) were calculated.
HPLC conditions
- Column:: Sumichiral OA, Sumitomo Chemical, 4.0 ⌀ x 250 mm
- Eluent solvent:: acetonitrile : H₂O = 25 : 75
- Flow rate:: 1 ml/min.
- Detector:: Hitachi UV detector L-4200, wavelength 254 nm
- Optical purity:: hydroxy compound 63.8%ee acetoxy compound 97.8%ee
- Conversion rate:: 60.5%

(1b) In 3780 ml of 1,4-dioxane was dissolved 4 g of ethyl 6,7-difluoro-1-acetoxymethyl-4-oxo-4H-[1,3]thiazeto[3,2-a]quinoline-3-carboxylate, a racemic compound, followed by addition of 220 ml of n-octyl alcohol. Then, 20 g of Lipase QL (Metito Sangyo) was added and the mixture was stirred at 30 °C for 27 hours for asymmetric alcoholysis. The resulting (-)-alcohol compound and (+)-acetoxy compound were determined using a chiral HPLC column and the respective optical purities and the rate of conversion (%) were calculated. The HPLC conditions were the same as described hereinbefore.
- Conversion rate:: 59.8%
- Optical purity:: acetoxy compound 98.3%ee(+)
alcohol compound 66.1%ee(-)
Then, the above reaction mixture was filtered through a 0.45 µ membrane filter (Millipore) to remove the enzyme and fractionally purified by the following process (2).
(2) The reaction mixture was concentrated under reduced pressure. The residue was purified by column chromatography (Wakogel C-200; chloroform : methanol = 100:1 → 10:1) to provide 1.57 g of (-)-hydroxy compound and 1.43 g of (+)-acetoxy compound.
(-)-Hydroxy compound: m. p. 285-286°C
Specific rotation [α] _{D} = -98.80° (DMF, c = 0.500)
(+)-Acetoxy compound: m. p. 250-251°C
Specific rotation [α] _{D} = +153.77° (DMF, c = 1.034)

| Elemental analysis for C₁₆H₁₃F₂NO₅S·1/3H₂O | | | |
|---|---|---|---|
| Calcd. (%): | C, 51.20; | H, 3.67; | N, 3.73 |
| Found (%): | C, 51.20; | H, 3.64; | N, 3.64 |

(3) Ethyl (+)-6,7-Difluoro-1-hydroxymethyl-4-oxo-4H-[1,3]thiazeto[3,2-a]quinoline-3-carboxylate
In 25 ml of ethanol was suspended 1.30 g of the (+)-acetoxy compound obtained in (2) above and the suspension was stirred at 80°C for 8 hours. The resulting precipitate was collected and washed with 50% ethanol/water to provide 0.95 g of (+)-hydroxy compound.
(+)-Hydroxy compound: m. p. 292-293°C (decomp.)
Specific rotation [α] _{D} = +145.09° (DMSO, c = 0.550)

| Elemental analysis for C₁₄H₁₁F₂NO₄S | | | |
|---|---|---|---|
| Calcd. (%): | C, 51.37; | H, 3.39; | N, 4.28 |
| Found (%): | C, 51.49; | H, 3.45; | N, 4.17 |

### Production Example 28b

### Ethyl 6,7-difluoro-1-hydroxymethyl-4-oxo-4H-[1,3]thiazeto[3,2-a]quinoline-3-carboxylate and ethyl 1-acetoxymethyl-6,7-difluoro-4-oxo-4H-[1,3]thiazeto[3,2-a]quinoline-3-carboxylate (production of optical isomers - Process B)

(1c) In 980 ml of tetrahydrofuran was dissolved 1 g of ethyl 1-acetoxymethyl-6,7-difluoro-4-oxo-4H-[1,3]thiazeto[3,2-a]quinoline-3-carboxylate, a racemic compound, followed by addition of 20 ml of ethanol. To this was added 15 g of Lipase PL (Meito Sangyo) and the mixture was stirred at 30°C for 59 hours for asymmetric alcoholysis. The resulting (+)-alcohol compound and (-)-acetoxy compound were determined using a chiral HPLC column and the respective optical purities and the rate of conversion (%) were calculated. The HPLC conditions were the same as described hereinbefore.
- Conversion rate:: 73.89%
- Optical purity:: acetoxy compound 99.51%ee(-) alcohol compound 35.17%ee(+)
Then, this reaction mixture was filtered through a 0.45 µ membrane filter (Millipore) to remove the enzyme, isolated and purified by the following process (2).
(2) The reaction mixture obtained in Production Example 28b (1) was treated as in Production Example 28a (2) to provide 259.4 mg of (-)-acetoxy compound and 427 mg of (+)-hydroxy compound.
(-)-Acetoxy compound: m. p. 251-252°C
Specific rotation [α] _{D} = -176.88° (DMF, c = 0.623)

| Elemental analysis for C₁₆H₁₃F₂NO₅S | | | |
|---|---|---|---|
| Calcd. (%): | C, 52.03; | H, 3.55; | N, 3.79 |
| Found (%): | C, 52.42; | H, 3.79; | N, 3.80 |

(+)-Hydroxy compound: m. p. 285°C (decomp.)
(3) Ethyl (-)-6,7-difluoro-1-hydroxymethyl-4-oxo-4H-[1,3]thiazeto[3,2-a]quinoline-3-carboxylate
The (-)-acetoxy compound obtained in (2) above was treated as in Production Example 28a (3) to provide (-)-hydroxy compound.
(-)-Hydroxy compound: m. p. 292-293°C (decomp.)
Specific rotation [α] _{D} = -156.55° (DMSO, c = 0.732)

### Production Example 29a

### n-Octyl 6,7-difluoro-1-hydroxymethyl-4-oxo-4H-[1,3]thiazeto[3,2-a]quinoline-3-carboxylate and ethyl 1-acetoxymethyl-6,7-difluoro-4-oxo-4H-[1,3]thiazeto[3,2-a]quinoline-3-carboxylate (production of optical isomers - Process C)

(1a) In 1 ml of THF was dissolved 1 mg of n-octyl 6,7-difluoro-1-hydroxymethyl-4-oxo-4H-[1,3]thiazeto[3,2-a]quinoline-3-carboxylate, a racemic compound, followed by addition of 20 µl of vinyl acetate and 2.5 mg of powdery molecular sieve. To this was added 20 mg of Lipozyme IM (Novo-Nordisk Bio Industries) and the mixture was stirred at 30°C for 25.6 hours for asymmetric esterification.
The resulting acetoxy compound and hydroxy compound were determined using a chiral HPLC column and the respective optical purities and the rate of conversion (%) were calculated.
HPLC conditions
- Column:: Sumichiral OA, Sumitomo Chemical, 4.0 ⌀ x 250 mm
- Eluent solvent:: acetonitrile : H₂O = 40 : 60
- Flow rate:: 1 ml/min.
- Detector:: Hitachi UV detector L-4200, wavelength 254 nm
- Optical purity:: (-)acetoxy compound 88.1%ee (+)-hydroxy compound 77.8%ee
- Conversion rate:: 46.9%

(1b) In 720 ml of tetrahydrofuran was dissolved 4 g of n-octyl 6,7-difluoro-1-hydroxymethyl-4-oxo-4H-[1,3]thiazeto[3,2-a]quinoline-3-carboxylate, a racemic compound, followed by addition of 80 ml of vinyl acetate and 8 g of powdery molecular sieve (Nacalai Tesque). To this was added 80 g of Lipozyme IM (Novo-Nordisk Bio Industries) and the mixture was stirred at 30°C for 8 hours for asymmetric esterification. The resulting (-)-acetoxy compound and (+)-hydroxy compound were determined under the same conditions as (1a) and the respective optical purities and the rate of conversion (%) were calculated.
- Optical purity:: (-)-acetoxy compound 73.1%ee (+)-hydroxy compound 99.5%ee
- Conversion rate:: 57.6%
Then, this reaction mixture was filtered through a 0.45 µ membrane filter (Millipore) to remove the enzyme followed by isolation and purification by the method described in (2). (2) The reaction mixture was concentrated under reduced pressure and the residue was dissolved in chloroform-methanol (5:1). Then, 20 g of silica gel (Wakogel C-200) was added and the above solution was concentrated under reduced pressure. The concentrate was adsorbed on a column (Wakogel C-200) and eluted with chloroform-methanol (100:1 → 50:1). Fraction 1 was concentrated and the resulting crystals were recrystallized from methanol and dried in vacuo to provide 1.63 g of hydroxy compound. Fraction 2 was also concentrated and the resulting crystals were washed with ether and dried in vacuo to provide 1.49 g of acetoxy compound.
(+)-Hydroxy compound: m. p. 251-252°C
Specific rotation [α] _{D} = +101.88° (DMF, c = 0.689)

| Elemental analysis for C₂₀H₂₃F₂NO₄S | | | |
|---|---|---|---|
| Calcd. (%): | C, 58.38; | H, 5.63; | N, 3.40 |
| Found (%): | C, 58.69; | H, 5.71; | N, 3.50 |

(-)-Acetoxy compound: m. p. 112-113°C
Specific rotation [α] _{D} = -107.95° (DMF, c = 0.830)

| Elemental analysis for C₂₂H₂₃F₂NO₅S | | | |
|---|---|---|---|
| Calcd. (%): | C, 58.27; | H, 5.56; | N, 3.09 |
| Found (%): | C, 58.32; | H, 5.35; | N, 3.13 |

### Production Example 29b

### n-Butyl 6,7-difluoro-1-hydroxymethyl-4-oxo-4H-[1,3]thiazeto[3,2-a]quinoline-3-carboxylate and n-butyl 1-acetoxymethyl-6,7-difluoro-4-oxo-4H-[1,3]thiazeto[3,2-a]quinoline-3-carboxylate (production of optical isomers - Process C)

(1) In 686 ml of tetrahydrofuran was dissolved 700 mg of n-butyl 6,7-difluoro-1-hydroxymethyl-4-oxo-4H-[1,3]thiazeto[3,2-a]quinoline-3-carboxylate, a racemic compound, followed by addition of 14 ml of vinyl acetate and 1.75 g of powdery molecular sieve (4A) (Nacalai Tesque). To this was added 21 g of Lipase PL (Meito Sangyo) and the mixture was stirred at 30°C for 3.5 hours for asymmetric esterification. The resulting (+)-acyl compound and (-)-hydroxy compound were determined using a chiral HPLC column and the respective optical purities and the rate of conversion (%) were calculated.
HPLC conditions
- Column:: Sumichiral OA, Sumitomo Chemical, 4.0 ⌀ x 250 mm
- Eluent solvent:: acetonitrile : H₂O = 35 : 65
- Flow rate:: 1 ml/min.
- Detector:: Hitachi UV detector L-4200, wavelength 254 nm
- Optical purity:: (+)-acetoxy compound 26.1%ee (-)-hydroxy compound 98.3%ee
- Conversion rate:: 79.0%
Then, this reaction mixture was filtered through a 0.45 µ membrane filter (Millipore) to remove the enzyme and followed by isolation and purification by the following process (2).
(2) The racemic compound obtained in Production Example 29b (1) was treated in the same manner as Production Example 29a (2) to provide 490 mg of (+)-acetoxy compound and 120 mg of (-)-hydroxy compound. (+)-Acetoxy compound: m. p. 184-186°C
Specific rotation [α] _{D} = +42.22° (DMF, c = 0.990)

| Elemental analysis for C₁₈H₁₇F₂NO₅S | | | |
|---|---|---|---|
| Calcd. (%): | C, 54.40; | H, 4.31; | N, 3.52 |
| Found (%): | C, 54.50; | H, 4.37; | N, 3.50 |

(-)-Hydroxy compound: m. p. 271-274°C
Specific rotation [α] _{D} = -133.74° (DMF, c = 0.966)

| Elemental analysis for C₁₆H₁₅F₂NO₄S | | | |
|---|---|---|---|
| Calcd. (%): | C, 54.08; | H, 4.25; | N, 3.94 |
| Found (%): | C, 54.21; | H, 4.16; | N, 3.86 |

### Production Example 30

### n-Octyl 1-acetoxymethyl-6,7-difluoro-4-oxo-4H-[1,3]thiazeto[3,2-a]quinoline-3-carboxylate and n-octyl 6,7-difluoro-1-hydroxymethyl-4-oxo-4H-[1,3]thiazeto[3,2-a]quinoline-3-carboxylate (production of optical isomers - Process D)

(1) In 180 ml of tetrahydrofuran was dissolved 1 g of the acetoxy compound obtained in Production Example 29a (2) above, followed by addition of 20 ml of ethanol. To this was added 20 g of Lipase PL (Meito Sangyo) and the mixture was stirred at 30°C for 51 hours for asymmetric alcoholysis. The resulting (+)-alcohol compound and (-)-acetoxy compound were determined using an optical resolution HPLC column and the respective optical purities and the rate of conversion (%) were calculated. The HPLC conditions were the same as used in Production Example 29 (1a).
- Optical purity:: (+)-hydroxy compound 50.4%ee, (-)-acetoxy compound 100%ee
- Conversion rate:: 35.2%

(2) Then, this reaction mixture was filtered through a 0.45 µ membrane filter (Millipore) to remove the enzyme and followed by isolation and purification by the method described in production Example 28 (2). As a result, 610 mg of the desirable compound was obtained as colorless powder. m. p. 124-126°C
Specific rotation [α] _{D} = -146.06° (DMF, c = 0.990)

| Elemental analysis for C₂₂H₂₅F₂NO₅S | | | |
|---|---|---|---|
| Calcd. (%): | C, 58.27; | H, 5.56; | N, 3.09 |
| Found (%): | C, 57.95; | H, 5.81; | N, 3.01 |

### Production Example 31

### Ethyl 6,7-difluoro-1-hydroxymethyl-4-oxo-4H-[1,3]thiazeto[3,2-a]quinoline-3-carboxylate (transesterification of optical isomer)

A suspension of 1.20 g of n-octyl (+)-6,7-difluoro-1-hydroxymethyl-4-oxo-4H-[1,3]thiazeto[3,2-a]quinoline-3-carboxylate in 1.0 % sulfuric acid-ethanol was stirred at 80 °C for 15 hours and the reaction mixture was worked up in the same manner as in Production Example 12 to provide 740 mg of the desirable compound. m. p. 293-296°C (decomp.)

In optical rotation, NMR, IR and elemental analysis, this product was identical with the compound obtained in Production Example 27 (4).

Optical isomers of the following compounds can be provided by the same procedure as Production Example 1-31.
n-Butyl 6,7-difluoro-1-fluoromethyl-4-oxo-4H-[1,3]thiazeto[3,2-a]quinoline-3-carboxylate
n-Amyl 6,7-difluoro-1-fluoromethyl-4-oxo-4H-[1,3]thiazeto[3,2-a]quinoline-3-carboxylate
n-Octyl 6,7-difluoro-1-fluoromethyl-4-oxo-4H-[1,3]thiazeto[3,2-a]quinoline-3-carboxylate

### Reference Example 1

### Ethyl 4-benzoyloxy-2-(1-chloro-2-fluoroethyl)thio-6-fluoro-7-(4-trifluoroacetylaminophenyl)quinoline-3-carboxylate

Using ethyl 6-fluoro-4-hydroxy-2-methoxymethylthio-7-(4-trifluoroacetylaminophenyl)quinoline-3-carboxylate, the procedure of Production Example 1 (1)-(4) was otherwise repeated to provide the desirable compound as light-yellow solid.

### Reference Example 2

### Ethyl 4-benzoyloxy-2-(1-chloro-2-fluoroethyl)thio-6-fluoro-7-phenylquinoline-3-carboxylate

Using ethyl 6-fluoro-4-hydroxy-2-methoxymethylthio-7-phenylquinoline-3-carboxylate, the procedure of Reference Example 1 was otherwise repeated to provide the desirable compound.
m. p. 145°C

### Reference Example 3

### Ethyl 6,7-difluoro-1-difluoromethyl-4-oxo-4H-[1,3]thiazeto[3,2-a]quinoline-3-carboxylate

Using ethyl 4-benzoyloxy-6,7-difluoro-2-mercaptoquinoline-3-carboxylate obtained in Production Example 1 (2) and 1-bromo-2,2-difluoroethane, the procedure of Production Example 1 (3)-(5) was otherwise repeated to provide the desirable compound. m. p. 184°C

### Reference Example 4

### Ethyl 4-benzoyloxy-2-(1-chloro-2-fluoroethyl)thio-6-fluoro-7-(4-trifluoroacetylaminomethylphenyl)quinoline-3-carboxylate

Using ethyl 6-fluoro-4-hydroxy-2-methoxymethylthio-7-(4-trifluoroacetylaminomethylphenyl)quinoline-3-carboxylate, the procedure of Production Example 1 (1)-(4) was otherwise repeated to provide the desirable compound. m. p. 188-189°C

### Example 1

### Ethyl 6-fluoro-1-fluoromethyl-4-oxo-7-(1-piperazinyl)-4H-[1,3]thiazeto[3,2-a]quinoline-3-carboxylate

A suspension prepared from 8.2 g of the ethyl 6,7-difluoro-1-fluoromethyl-4-oxo-4H-[1,3]thiazeto[3,2-a]quinoline-3-carboxylate obtained in Production Example 1, 6.88 g of potassium carbonate, 80 ml of N,N-dimethylformamide, and 4.29 g of piperazine was stirred at 60°C for 3 hours. This reaction mixture was concentrated under reduced pressure and the residue was diluted with 100 ml of water and stirred for a while. The crystals that separated out were collected by filtration, washed with water, ethanol and ether, and dried to provide 2.94 g of the desirable compound. m. p. 187°C

| Elemental analysis for C₁₈H₁₉F₂N₃O₃S·1/2H₂O | | | |
|---|---|---|---|
| Calcd. (%): | C, 53.46; | H, 4.98; | N, 10.39 |
| Found (%): | C, 53.55; | H, 4.94; | N, 10.45 |

### Example 2

### 6-Fluoro-1-methylene-4-oxo-7-(1-piperazinyl)-4H-[1,3]thiazeto[3,2-a]quinoline-3-carboxylic acid

A suspension of 1.90 g of the ethyl 6-fluoro-1-fluoromethyl-4-oxo-7-(1-piperazinyl)-4H-[1,3]thiazeto[3,2-a]quinoline-3-carboxylate obtained in Example 1 in 100 ml of 1% potassium hydroxide solution in tert-butanol-water was stirred at ambient temperature for 54 hours. From this reaction mixture, the insoluble matter was filtered off and the mother liquor was neutralized with acetic acid. The resulting crystals were collected by filtration, washed with ethanol and ether, and dried to provide 1.6 g of the desirable compound. m. p. ³300°C (decomp.)

| Elemental analysis for C₁₆H₁₄FN₃O₃S·H₂O | | | |
|---|---|---|---|
| Calcd. (%): | C, 52.60; | H, 4.41; | N, 11.50 |
| Found (%): | C, 52.33; | H, 4.23; | N, 11.39 |

IR cm⁻¹ 3400, 3000, 1720, 1610, 1495, 1360, 1250, 1220, 1100, 820, 790

### Example 3

### 6-Fluoro-1-methylene-7-[4-(5-methyl-2-oxo-1,3-dioxol-4-yl)methyl-1-piperazinyl]-4-oxo-4H-[1,3]thiazeto[3,2-a]quinoline-3-carboxylic acid

A suspension consisting of 180 mg of the 6-fluoro-1-methylene-7-(1-piperazinyl)-4-oxo-4H-[1,3]thiazeto[3,2-a]quinoline-3-carboxylic acid obtained in Example 2, 98 mg of potassium hydrogen carbonate, 3 ml of N,N-dimethylformamide and 0.79 g of 4-bromomethyl-5-methyl-1,3-dioxol-2-one in acetone was stirred at ambient temperature for 3 hours. To this reaction mixture was added 10 ml of water and the resulting crystals were collected by filtration, washed with water, ethanol and ether, and dried. The powder obtained was dissolved in 100 ml of chloroform-methanol and the insoluble matter was filtered off. The filtrate was concentrated to 10 ml and the resulting crystals were collected by filtration, washed with ether, and dried to provide 70 mg of the desirable compound as light-yellow solid.
m. p. 199°C

| Elemental analysis for C₂₁H₁₈FN₃O₆S | | | |
|---|---|---|---|
| Calcd. (%): | C, 54.90; | H, 3.95; | N, 9.15 |
| Found (%): | C, 54.60; | H, 4.20; | N, 9.52 |

### Example 4

### Ethyl 6-fluoro-1-fluoromethyl-4-oxo-7-(4-trifluoroacetylaminophenyl)-4H-[1,3]thiazeto[3,2-a]quinoline-3-carboxylate

A solution consisting of 4.0 g of ethyl 4-benzoyloxy-2-(1-chloro-2-fluoroethyl)thio-6-fluoro-7(4-trifluoroacetylaminophenyl)quinoline-3-carboxylate, 40 ml of tetrahydrofuran and 3.17 g of triethylamine was refluxed at a bath temperature not over 80°C for 156 hours. The reaction mixture was then cooled and the resulting crystals were collected by filtration and washed with tetrahydrofuran to give a gray powder. This powder was dissolved in about 1 l of chloroform-methanol, washed with water, dried over magnesium sulfate, and concentarted. The residue was purified by column chromatography to provide 2.9 g of the desirable compound. m. p. 272°C

| Elemental analysis for C₂₂H₁₅F₅N₂O₄S | | | |
|---|---|---|---|
| Calcd. (%): | C, 53.01; | H, 3.03; | N, 5.62 |
| Found (%): | C, 53.39; | H, 3.41; | N, 5.75 |

### Example 5

### 7-(4-aminophenyl)-6-fluoro-1-methylene-4-oxo-4H-[1,3]thiazeto[3,2-a]quinoline-3-carboxylic acid

In 25 ml of 1% potassium hydroxyde in t-butanol-water was suspended 500 mg of the compound obtained in Example 4 and the reaction was conducted at ambient temperature for 7 hours and further at 50°C for 2 hours. The reaction mixture was then neutralized with acetic acid, diluted with 50 ml of water, and extracted with chloroform-methanol. The extract was washed with water, dried over magnesium sulfate, and concentrated to provide 270 mg of the desirable compound as yellow solid. m. p. ³300°C (decomp.)

| Elemental analysis for C₁₈H₁₁FN₂O₃S·3/4H₂O | | | |
|---|---|---|---|
| Calcd. (%): | C, 58.77; | H, 3.42; | N, 7.61 |
| Found (%): | C, 58.92; | H, 3.21; | N, 7.26 |

IR cm⁻¹ 3450, 3350, 1920, 1630, 1490, 1360, 1290, 1190, 1115, 1020, 790
The following compounds were synthesized in the same manner as Examples 1-5.

### Example 6

### Ethyl 6,8-difluoro-1-fluoromethyl-4-oxo-7-(1-piperazinyl)-4H-[1,3]thiazeto[3,2-a]quinoline-3-carboxylate

m. p. 259°C

| Elemental analysis for C₁₈H₁₈F₃N₃O₃S·H₂O | | | |
|---|---|---|---|
| Calcd. (%): | C, 50.11; | H, 4.67; | N, 9.74 |
| Found (%): | C, 50.38; | H, 4.61; | N, 9.63 |

### Example 7

### Ethyl 6-fluoro-1-fluoromethyl-4-oxo-7-phenyl-4H-[1,3]thiazeto[3,2-a]quinoline-3-carboxylate

m. p. 241-242°C

| Elemental analysis for C₂₀H₁₅F₂NO₃S | | | |
|---|---|---|---|
| Calcd. (%): | C, 62.00; | H, 3.90; | N, 3.62 |
| Found (%): | C, 61.70; | H, 3.87; | N, 3.65 |

### Example 8

### 6-Fluoro-1-methylene-4-oxo-7-phenyl-4H-[1,3]thiazeto[3,2-a]quinoline-3-carboxylic acid

m. p. 265-266°C (decomp.)

| Elemental analysis for C₁₈H₁₀FNO₃S | | | |
|---|---|---|---|
| Calcd. (%): | C, 63.71; | H, 2.97; | N, 4.13 |
| Found (%): | C, 63.40; | H, 3.14; | N, 4.03 |

### Example 9

### Ethyl 7-(4-aminopiperidino)-6-fluoro-1-fluoromethyl-4-oxo-4H-[1,3]thiazeto[3,2-a]quinoline-3-carboxylate

m. p. 230°C (decomp.)

| Elemental analysis for C₁₉H₂₁F₂N₃O₃S·1/2H₂O | | | |
|---|---|---|---|
| Calcd. (%): | C, 54.53; | H, 5.30; | N, 10.04 |
| Found (%): | C, 54.14; | H, 5.00; | N, 9.67 |

### Example 10

### Ethyl 7-(3-amino-1-pyrrolidinyl)-6-fluoro-1-methylene-4-oxo-4H-[1,3]thiazeto[3,2-a]quinoline-3-carboxylate

m. p. ³300°C (decomp.)

| Elemental analysis for C₁₈H₁₈FN₃O₃S | | | |
|---|---|---|---|
| Calcd. (%): | C, 57.59; | H, 4.83; | N, 11.19 |
| Found (%): | C, 58.82; | H, 4.90; | N, 11.27 |

IR cm⁻¹ 3350, 2900, 1720, 1630, 1595, 1510, 1370, 1320, 1100, 890, 800

### Example 11

### Ethyl 6-fluoro-1-fluoromethyl-7-morpholino-4-oxo-4H-[1,3]thiazeto[3,2-a]quinoline-3-carboxylate

m. p. 273-274°C

| Elemental analysis for C₁₈H₁₈F₂N₂O₄S | | | |
|---|---|---|---|
| Calcd. (%): | C, 54.54; | H, 4.58; | N, 7.07 |
| Found (%): | C, 54.54; | H, 4.58; | N, 7.07 |

### Example 12

### Ethyl 6-fluoro-1-fluoromethyl-7-(4-methyl-1-piperazinyl)-4-oxo-4H-[1,3]thiazeto[3,2-a]quinoline-3-carboxylate

m. p. 251°C

| Elemental analysis for C₁₉H₂₁F₂N₃O₃S | | | |
|---|---|---|---|
| Calcd. (%): | C, 55.73; | H, 5.17; | N, 10.26 |
| Found (%): | C, 55.62; | H, 5.28; | N, 10.10 |

### Example 13

### 6-Fluoro-1-methylene-7-(4-methyl-1-piperazinyl)-4-oxo-4H-[1,3]thiazeto[3,2-a]quinoline-3-carboxylic acid

m. p. 207°C

| Elemental analysis for C₁₇H₁₆FN₃O₃S·H₂O | | | |
|---|---|---|---|
| Calcd. (%): | C, 53.81; | H, 4.78; | N, 11.08 |
| Found (%): | C, 54.02; | H, 4.37; | N, 11.65 |

### Example 14

### Ethyl 6-fluoro-1-fluoromethyl-4-oxo-7-(1-pyrrolidinyl)-4H-[1,3]thiazeto[3,2-a]quinoline-3-carboxylate

m. p. 274°C

| Elemental analysis for C₁₈H₁₈F₂N₂O₃S | | | |
|---|---|---|---|
| Calcd. (%): | C, 56.83; | H, 4.77; | N, 7.36 |
| Found (%): | C, 56.60; | H, 4.72; | N, 7.05 |

### Example 15

### 6-Fluoro-1-methylene-4-oxo-7-(1-pyrrolidinyl)-4H-[1,3]thiazeto[3,2-a]quinoline-3-carboxylic acid

m. p. 285-286°C (decomp.)

| Elemental analysis for C₁₆H₁₃FN₂O₃S·3/2H₂O | | | |
|---|---|---|---|
| Calcd. (%): | C, 53.47; | H, 4.49; | N, 7.79 |
| Found (%): | C, 53.41; | H, 4.04; | N, 7.54 |

### Example 16

### Ethyl 6-fluoro-1-fluoromethyl-8-methoxy-4-oxo-7-(1-piperazinyl)-4H-[1,3]thiazeto[3,2-a]quinoline-3-carboxylate

m. p. 173-175°C (decomp.)

| Elemental analysis for C₁₉H₂₁F₂N₃O₄S·1/2H₂O | | | |
|---|---|---|---|
| Calcd. (%): | C, 52.52; | H, 5.10; | N, 9.67 |
| Found (%): | C, 52.31; | H, 5.04; | N, 9.28 |

### Example 17

### Ethyl 6-fluoro-1-fluoromethyl-8-methoxy-7-(4-methyl-1-piperazinyl)-4-oxo-4H-[1,3]thiazeto[3,2-a]quinoline-3-carboxylate

In 2.5 ml of 35% formaldehyde was dissolved 252.9 mg of the compound obtained in Example 16, and the solution and 1.3 ml of formic acid were refluxed for 30 minutes. After cooling, the reaction mixture was poured over aqueous solution of sodium hydrogen carbonate and extracted with chloroform. The organic layer was dried and concentrated under reduced pressure to give 229 mg of solid. This solid was purified by silica gel column chromatography (eluent-methanol : chloroform = 2 : 98) to provide 179.2 mg of the desirable compound.
m. p. 212-213°C (decomp.)

| Elemental analysis for C₂₀H₂₃F₂N₃O₄S | | | |
|---|---|---|---|
| Calcd. (%): | C, 54.66; | H, 5.27; | N, 9.56 |
| Found (%): | C, 54.82; | H, 4.87; | N, 9.66 |

### Example 18

### 6-Fluoro-1-fluoromethyl-8-methoxy-7-(4-methyl-1-piperazinyl)-4-oxo-4H-[1,3]thiazeto[3,2-a]quinoline-3-carboxylic acid

The compound of Example 17, 87.4 mg, was hydrolyzed using fuming sulfuric acid in the per se conventional manner to provide 32.7 mg of the desirable compoud.
m. p. 200-201°C (decomp.)

| Elemental analysis for C₁₈H₁₉F₂N₃O₄S | | | |
|---|---|---|---|
| Calcd. (%): | C, 52.55; | H, 4.65; | N, 10.21 |
| Found (%): | C, 52.32; | H, 4.34; | N, 10.21 |

The following compounds were synthesized in the same manner as Examples 1-18.

### Example 19

### Ethyl 6-fluoro-1-fluoromethyl-4-oxo-7-piperidino-4H-[1,3]thiazeto[3,2-a]quinoline-3-carboxylate

m. p. 244-245°C

| Elemental analysis for C₁₉H₂₀F₂N₂O₃S | | | |
|---|---|---|---|
| Calcd. (%): | C, 57.86; | H, 5.11; | N, 7.10 |
| Found (%): | C, 57.86; | H, 5.13; | N, 7.02 |

### Example 20

### 6-Fluoro-1-methylene-4-oxo-7-piperidino-4H-[1,3]thiazeto[3,2-a]quinoline-3-carboxylic acid

m. p. 246°C (decomp.)

| Elemental analysis for C₁₇H₁₅FN₂O₃S | | | |
|---|---|---|---|
| Calcd. (%): | C, 55.73; | H, 4.40; | N, 7.65 |
| Found (%): | C, 56.37; | H, 4.01; | N, 7.58 |

### Example 21

### 6-Fluoro-1-methylene-7-morpholino-4-oxo-4H-[1,3]thiazeto[3,2-a]quinoline-3-carboxylic acid

m. p. 276-277°C (decomp.)

| Elemental analysis for C₁₆H₁₃FN₂O₄S·H₂O | | | |
|---|---|---|---|
| Calcd. (%): | C, 52.45; | H, 4.13; | N, 7.65 |
| Found (%): | C, 52.64; | H, 3.98; | N, 7.66 |

### Example 22

### Ethyl 6,8-difluoro-1-fluoromethyl-7-(4-methyl-1-piperazinyl)-4-oxo-4H-[1,3]thiazeto[3,2-a]quinoline-3-carboxylate

m. p. 217°C

| Elemental analysis for C₁₉H₂₀F₃N₃O₃S | | | |
|---|---|---|---|
| Calcd. (%): | C, 53.39; | H, 4.72; | N, 9.83 |
| Found (%): | C, 53.26; | H, 4.64; | N, 9.95 |

### Example 23

### 6,8-Difluoro-1-fluoromethyl-7-(4-methyl-1-piperazinyl)-4-oxo-4H-[1,3]thiazeto[3,2-a]quinoline-3-carboxylic acid

m. p. 226°C

| Elemental analysis for C₁₇H₁₆F₃N₃O₃S | | | |
|---|---|---|---|
| Calcd. (%): | C, 51.12; | H, 4.04; | N, 10.52 |
| Found (%): | C, 51.40; | H, 3.89; | N, 10.33 |

### Example 24

### Ethyl 6,8-difluoro-1-methylene-7-(4-methyl-1-piperazinyl)-4-oxo-4H-[1,3]thiazeto[3,2-a]quinoline-3-carboxylate

m. p. 151°C

| Elemental analysis for C₁₉H₁₉F₂N₃O₃S | | | |
|---|---|---|---|
| Calcd. (%): | C, 56.01; | H, 4.70; | N, 10.32 |
| Found (%): | C, 55.75; | H, 5.02; | N, 10.54 |

### Example 25

### Ethyl 6-fluoro-1-fluoromethyl-7-(3-methyl-1-piperazinyl)-4-oxo-4H-[1,3]thiazeto[3,2-a]quinoline-3-carboxylate

m. p. 214°C

| Elemental analysis for C₁₉H₂₁F₂N₃O₃S | | | |
|---|---|---|---|
| Calcd. (%): | C, 55.73; | H, 5.17; | N, 10.26 |
| Found (%): | C, 55.50; | H, 5.12; | N, 10.10 |

### Example 26

### 7-[(3R)-3-amino-1-pyrrolidinyl]-6-fluoro-1-methylene-4-oxo-4H-[1,3]thiazeto[3,2-a]quinoline-3-carboxylic acid

Using (3R)-3-amino-1-pyrrolidine and ethyl 6,7-difluoro-1-fluoromethyl-4-oxo-4H-[1,3]thiazeto[3,2-a]quinoline-3-carboxylate, the desirable compound was obtained in the same manner as Examples 1 and 2.
m. p. ³300°C (decomp.)

| Elemental analysis for C₁₆H₁₄FN₃O₃S·H₂O | | | |
|---|---|---|---|
| Calcd. (%): | C, 52.60; | H, 4.41; | N, 11.50 |
| Found (%): | C, 52.42; | H, 4.43; | N, 11.16 |

IR (cm⁻¹) 3400, 3000, 1630, 1510, 1360, 1230, 790 Example 27 7-[(3S)-3-amino-1-pyrrolidinyl]-6-fluoro-1-methylene-4-oxo-4H-[1,3]thiazeto[3,2-a]quinoline-3-carboxylic acid
Using (3S)-3-amino-1-pyrrolidine and ethyl 6,7-difluoro-1-fluoromethyl-4-oxo-4H-[1,3]thiazeto[3,2-a]quinoline-3-carboxylate, the procedure of Example 26 was otherwise repeated to provide the desirable compound.
m. p. ³300°C (decomp.)

| Elemental analysis for C₁₆H₁₄FN₃O₃S·H₂O | | | |
|---|---|---|---|
| Calcd. (%): | C, 52.60; | H, 4.41; | N, 11.50 |
| Found (%): | C, 52.90; | H, 4.80; | N, 11.87 |

IR (cm⁻¹) 3400, 3000, 1630, 1510, 1360, 1230, 790
The following compounds were synthesized in the same manner as Examples 1-27.

### Example 28

### 6-Fluoro-1-methylene-7-(3-methyl-1-piperazinyl)-4-oxo-4H-[1,3]thiazeto[3,2-a]quinoline-3-carboxylic acid

m. p. 285°C

| Elemental analysis for C₁₇H₁₆FN₃O₃S | | | |
|---|---|---|---|
| Calcd. (%): | C, 50.24; | H, 5.21; | N, 10.34 |
| Found (%): | C, 50.28; | H, 4.88; | N, 10.16 |

### Example 29

### 6-Fluoro-1-fluoromethyl-7-(4-methyl-1-piperazinyl)-4-oxo-4H-[1,3]thiazeto[3,2-a]quinoline-3-carboxylic acid

m. p. 232-235°C (decomp.)

| Elemental analysis for C₁₇H₁₇F₂N₃O₃S | | | |
|---|---|---|---|
| Calcd. (%): | C, 53.09; | H, 4.36; | N, 10.83 |
| Found (%): | C, 53.31; | H, 4.13; | N, 10.73 |

### Example 30

### 6-Fluoro-1-fluoromethyl-7-morpholino-4-oxo-4H-[1,3]thiazeto[3,2-a]quinoline-3-carboxylic acid

m. p. 239°C

| Elemental analysis for C₁₆H₁₄F₂N₂O₄S | | | |
|---|---|---|---|
| Calcd. (%): | C, 52.17; | H, 3.83; | N, 7.60 |
| Found (%): | C, 52.61; | H, 3.73; | N, 7.51 |

### Example 31

### 7-[(3S)-3-Amino-1-pyrrolidinyl]-6-fluoro-1-fluoromethyl-4-oxo-4H-[1,3]thiazeto[3,2-a]quinoline-3-carboxylic acid

m. p. 265-270°C (decomp.)

| Elemental analysis for C₁₆H₁₅F₂N₃O₃S·2H₂O | | | |
|---|---|---|---|
| Calcd. (%): | C, 47.64; | H, 4.75; | N, 10.42 |
| Found (%): | C, 47.43; | H, 4.84; | N, 10.38 |

### Example 32

### 7-(cis-3,5-Dimethyl-1-piperazinyl)-6-fluoro-1-fluoromethyl-4-oxo-4H-[1,3]thiazeto[3,2-a]quinoline-3-carboxylic acid

m. p. 220°C

| Elemental analysis for C₁₈H₁₉F₂N₃O₃S·H₂O | | | |
|---|---|---|---|
| Calcd. (%): | C, 52.29; | H, 5.12; | N, 10.16 |
| Found (%): | C, 52.44; | H, 5.48; | N, 10.38 |

### Example 33

### 8-Chloro-6-fluoro-1-fluoromethyl-7-(4-methyl-1-piperazinyl)-4-oxo-4H-[1,3]thiazeto[3,2-a]quinoline-3- carboxylic acid hydrochloride

In 3 ml of methylene chloride were suspended 320 mg of 6-fluoro-1-fluoromethyl-7-(4-methyl-1-piperazinyl)-4-oxo-4H-[1,3]thiazeto[3,2-a]quinoline-3-carboxylic acid and 125 mg of sulfinyl chloride and the reaction was conducted at ambient temperature for 3 hours. From this reaction mixture, the crystals separated out were filtered, and washed serially with ethanol and ether and dried to provide 250 mg of the desirable compound as light-brown powder.
m. p. 230-235°C (decomp.)

| Elemental analysis for C₁₇H₁₆ClF₂N₃O₃S·HCl·1/2H₂O | | | |
|---|---|---|---|
| Calcd. (%): | C, 44.26; | H, 3.71; | N, 9.11 |
| Found (%): | C, 44.10; | H, 3.68; | N, 8.93 |

The following compounds were synthesized in the same manner as Examples 1-33.

### Example 34

### 6-Fluoro-1-fluoromethyl-7-(4-hydroxypiperidino)-4-oxo-4H-[1,3]thiazeto[3,2-a]quinoline-3-carboxylic acid

m. p. 245-248°C (decomp.)

| Elemental analysis for C₁₇H₁₆F₂N₂O₄S | | | |
|---|---|---|---|
| Calcd. (%): | C, 53.40; | H, 4.22; | N, 7.33 |
| Found (%): | C, 53.58; | H, 4.00; | N, 7.60 |

### Example 35

### 6-Fluoro-1-fluoromethyl-7-thiomorpholin-4-oxo-4H-[1,3]thiazeto[3,2-a]quinoline-3-carboxylic acid

m. p. 231-234°C (decomp.)

| Elemental analysis for C₁₆H₁₄F₂N₂O₃S₂ | | | |
|---|---|---|---|
| Calcd. (%): | C, 49.99; | H, 3.67; | N, 7.29 |
| Found (%): | C, 50.29; | H, 3.86; | N, 7.29 |

### Example 36

### Ethyl 1-difluoromethyl-6-fluoro-7-(4-methyl-1-piperazinyl)-4-oxo-4H-[1,3]thiazeto[3,2-a]quinoline-3-carboxylate

m. p. 232°C

| Elemental analysis for C₁₉H₂₀F₃N₃O₃S | | | |
|---|---|---|---|
| Calcd. (%): | C, 53.39; | H, 4.27; | N, 9.83 |
| Found (%): | C, 53.49; | H, 4.43; | N, 9.75 |

### Example 37

### 6-Fluoro-1-fluoromethyl-7-(4-hydroxymethylpiperidino)-4-oxo-4H-[1,3]thiazeto[3,2-a]quinoline-3-carboxylic acid

m. p. 234-236°C (decomp.)

| Elemental analysis for C₁₈H₁₈F₂N₂O₄S | | | |
|---|---|---|---|
| Calcd. (%): | C, 54.54; | H, 4.58; | N, 7.07 |
| Found (%): | C, 54.53; | H, 4.81; | N, 7.16 |

### Example 38

### 6-Fluoro-1-fluoromethyl-4-oxo-7-(1-piperazinyl)-4H-[1,3]thiazeto[3,2-a]quinoline-3-carboxylic acid hydrochloride

m. p. 270-272°C (decomp.)

| Elemental analysis for C₁₆H₁₅F₂N₃O₃S·HCl | | | |
|---|---|---|---|
| Calcd. (%): | C, 47.59; | H, 3.74; | N, 10.41 |
| Found (%): | C, 47.70; | H, 3.95; | N, 10.34 |

### Example 39

### 7-(4-Aminopiperidino)-6-fluoro-1-fluoromethyl-4-oxo-4H-[1,3]thiazeto[3,2-a]quinoline-3-carboxylic acid hydrochloride

m. p. ³300°C (decomp.)

| Elemental analysis for C₁₇H₁₇F₂N₃O₃S·HCl·H₂O | | | |
|---|---|---|---|
| Calcd. (%): | C, 46.84; | H, 4.62; | N, 9.64 |
| Found (%): | C, 47.02; | H, 4.56; | N, 9.46 |

IR (cm⁻¹) 2950, 1700, 1630, 1600, 1510, 1390, 1260, 800

### Example 40

### 7-(4-Aminomethylpiperidino)-6-fluoro-1-fluoromethyl-4-oxo-4H-[1,3]thiazeto[3,2-a]quinoline-3-carboxylic acid hydrochloride

m. p. 253-257°C (decomp.)

| Elemental analysis for C₁₈H₁₉F₂N₃O₃S·HCl | | | |
|---|---|---|---|
| Calcd. (%): | C, 50.06; | H, 4.67; | N, 9.73 |
| Found (%): | C, 50.34; | H, 4.68; | N, 9.58 |

### Example 41

### 6-Fluoro-1-fluoromethyl-7-(3-hydroxy-1-azetidinyl)-4-oxo-4H-[1,3]thiazeto[3,2-a]quinoline-3-carboxylic acid

m. p. ³300°C (decomp.)

| Elemental analysis for C₁₅H₁₂F₂N₂O₄S·2H₂O·1/4iso-PrOH | | | |
|---|---|---|---|
| Calcd. (%): | C, 46.66; | H, 4.48; | N, 6.91 |
| Found (%): | C, 46.42; | H, 4.01; | N, 6.54 |

IR (cm⁻¹) 3300, 1700, 1630, 1540, 1450, 1390, 1240, 810

### Example 42

### 7-(cis-3,5-Dimethyl-1-piperazinyl)-6,8-difluoro-1-methylene-4-oxo-4H-[1,3]thiazeto[3,2-a]quinoline-3-carboxylic acid

m. p. ³300°C (decomp.)

| Elemental analysis for C₁₈H₁₇F₂N₃O₃S·1/2H₂O | | | |
|---|---|---|---|
| Calcd. (%): | C, 53.72; | H, 4.51; | N, 10.44 |
| Found (%): | C, 53.96; | H, 4.54; | N, 10.48 |

IR (cm⁻¹) 3600, 2750, 1630, 1470, 1430, 1100

### Example 43

### Ethyl 7-[3,7-diazabicyclo[3,3,0]oct-1(5)-en-3-yl]-6-fluoro-1-fluoromethyl-4-oxo-4H-[1,3]thiazeto[3,2-a]quinoline-3-carboxylate

m. p. 242-243°C (decomp.)

| Elemental analysis for C₂₀H₁₉F₂N₃O₃S·1/4H₂O | | | |
|---|---|---|---|
| Calcd. (%): | C, 56.66; | H, 4.64; | N, 9.91 |
| Found (%): | C, 56.41; | H, 4.33; | N, 9.94 |

### Example 44

### Ethyl 6-fluoro-1-fluoromethyl-7-(7-methyl-3,7-diazabicyclo[3,3,0]oct-1(5)-en-3-yl]-4-oxo-4H-[1,3]thiazeto[3,2-a]quinoline-3-carboxylate

m. p. 250-252°C (decomp.)

| Elemental analysis for C₂₁H₂₁F₂N₃O₃S·H₂O | | | |
|---|---|---|---|
| Calcd. (%): | C, 55.87; | H, 5.13; | N, 9.31 |
| Found (%): | C, 55.86; | H, 4.98; | N, 9.13 |

### Example 45

### Ethyl 7-[4,7-diazaspiro[2,5]octan-7-yl]-6-fluoro-1-fluoromethyl-4-oxo-4H-[1,3]thiazeto[3,2-a]quinoline-3-carboxylate

m. p. 198-200°C

| Elemental analysis for C₂₀H₂₁F₂N₃O₃S·3/2H₂O | | | |
|---|---|---|---|
| Calcd. (%): | C, 53.56; | H, 5.39; | N, 9.37 |
| Found (%): | C, 53.12; | H, 4.86; | N, 9.83 |

### Example 46

### Ethyl 6,8-difluoro-1-fluoromethyl-7-morpholino-4-oxo-4H-[1,3]thiazeto[3,2-a]quinoline-3-carboxylate

m. p. 227-228°C

| Elemental analysis for C₁₈H₁₇F₃N₂O₄S | | | |
|---|---|---|---|
| Calcd. (%): | C, 52.17; | H, 4.14; | N, 6.76 |
| Found (%): | C, 52.19; | H, 4.01; | N, 6.50 |

### Example 47

### Ethyl 7-(cis-3,5-dimethyl-1-piperazinyl)-6,8-difluoro-1-fluoromethyl-4-oxo-4H-[1,3]thiazeto[3,2-a]quinoline-3-carboxylate

m. p. 196-197°C

| Elemental analysis for C₂₀H₂₂F₃N₃O₃S | | | |
|---|---|---|---|
| Calcd. (%): | C, 54.41; | H, 5.02; | N, 9.52 |
| Found (%): | C, 54.68; | H, 4.73; | N, 9.34 |

### Example 48

### Ethyl 1-difluoromethyl-6-fluoro-7-morpholino-4-oxo-4H-[1,3]thiazeto[3,2-a]quinoline-3-carboxylate

m. p. 252°C

| Elemental analysis for C₁₈H₁₇F₃N₂O₄S | | | |
|---|---|---|---|
| Calcd. (%): | C, 52.17; | H, 4.14; | N, 6.76 |
| Found (%): | C, 52.02; | H, 4.23; | N, 6.94 |

### Example 49

### Ethyl 1-difluoromethyl-7-(cis-3,5-dimethyl-1-piperazinyl)-6-fluoro-4-oxo-4H-[1,3]thiazeto[3,2-a]quinoline-3-carboxylate

m. p. 239°C

| Elemental analysis for C₂₀H₂₂F₃N₃O₃S | | | |
|---|---|---|---|
| Calcd. (%): | C, 54.41; | H, 5.02; | N, 9.52 |
| Found (%): | C, 54.72; | H, 4.66; | N, 9.66 |

### Example 50

### Ethyl 8-chloro-6-fluoro-1-fluoromethyl-7-morpholino-4-oxo-4H-[1,3]thiazeto[3,2-a]quinoline-3-carboxylate

m. p. 253-254°C

| Elemental analysis for C₁₈H₁₇ClF₂N₂O₄S | | | |
|---|---|---|---|
| Calcd. (%): | C, 50.18; | H, 3.98; | N, 6.50 |
| Found (%): | C, 50.60; | H, 3.76; | N, 6.13 |

### Example 51

### Ethyl 6-fluoro-1-fluoromethyl-4-oxo-7-(4-trifluoroacetylaminomethylphenyl)-4H-[1,3]thiazeto[3,2-a]quinoline-3-carboxylate

m. p. 276-278°C

| Elemental analysis for C₂₃H₁₈F₅N₂O₄S | | | |
|---|---|---|---|
| Calcd. (%): | C, 53.80; | H, 3.53; | N, 5.46 |
| Found (%): | C, 53.83; | H, 3.21; | N, 5.43 |

### Example 52

### Ethyl 7-(4,7-diazaspiro[2,5]octan-7-yl)-6,8-difluoro-1-fluoromethyl-4-oxo-4H-[1,3]thiazeto[3,2-a]quinoline-3-carboxylate

m. p. 179-181°C

| Elemental analysis for C₂₀H₂₀F₃N₃O₃S·1/2H₂O | | | |
|---|---|---|---|
| Calcd. (%): | C, 53.56; | H, 4.72; | N, 9.37 |
| Found (%): | C, 53.58; | H, 4.75; | N, 9.70 |

### Example 53

### Ethyl 6-fluoro-1-fluoromethyl-7-(4-methyl-4,7-diazaspiro[2,5]octan-7-yl)-4-oxo-4H-[1,3]thiazeto[3,2-a]quinoline-3-carboxylate

m. p. 226-227°C

| Elemental analysis for C₂₁H₂₃F₂N₃O₃S·1/2H₂O | | | |
|---|---|---|---|
| Calcd. (%): | C, 55.61; | H, 5.56; | N, 9.26 |
| Found (%): | C, 55.20; | H, 5.49; | N, 9.80 |

### Example 54

### Ethyl 6,8-difluoro-1-fluoromethyl-7-(4-methyl-4,7-diazaspiro[2,5]octan-7-yl)-4-oxo-4H-[1,3]thiazeto[3,2-a]quinoline-3-carboxylate

m. p. 202-203°C

| Elemental analysis for C₂₁H₂₂F₃N₃O₃S·1/4H₂O | | | |
|---|---|---|---|
| Calcd. (%): | C, 55.07; | H, 4.95; | N, 9.18 |
| Found (%): | C, 54.74; | H, 4.59; | N, 9.59 |

### Example 55

### 6-Fluoro-1-fluoromethyl-7-(7-methyl-3,7-diazabicyclo[3,3,0]oct-1(5)-en-3-yl)-4-oxo-4H-[1,3]thiazeto[3,2-a]quinoline-3-carboxylic acid sulfate

m. p. 250°C (decomp.)

| Elemental analysis for C₁₉H₁₇F₂N₃O₃S·H₂SO₄·H₂O | | | |
|---|---|---|---|
| Calcd. (%): | C, 43.76; | H, 4.05; | N, 8.06 |
| Found (%): | C, 44.05; | H, 4.08; | N, 8.06 |

### Example 56

### Production of ethyl 6-fluoro-1-fluoromethyl-7-(4-methyl-1-piperazinyl)-4-oxo-4H-[1,3]thiazeto[3,2-a]quinoline-3-carboxylate

In 4 ml of methylene chloride was suspended 85.6 mg of ethyl 6-fluoro-1-hydroxymethyl-4-oxo-4H-[1,3]thiazeto[3,2-a]quinoline-3-carboxylate. To this suspension was added 86.0 mg of DAST at 0°C and the mixture was stirred at ambient temperature for 3 hours. This reaction mixture was poured in an ice-cooled saturated aqueous solution of sodium hydrogen carbonate and extracted with a solvent mixture of chloroform and methanol (4:1). The organic layer was dried and concentrated under reduced pressure and the residue was purified by silica gel column chromatography (chloroform : methanol = 95 : 5) to provide 27.2 mg of the desirable compound.
m. p. 249-251°C.

In elemental analysis, this product was identical with the compound obtained in Example 12.

### Example 57

### Production of ethyl (+)-6-fluoro-1-fluoromethyl-7-(4-methyl-1-piperazinyl)-4-oxo-4H-[1,3]thiazeto[3,2-a]quinoline-3-carboxylate (synthesis of optical isomer)

Using 345 mg of the optically active compound ethyl (+)-6,7-difluoro-1-fluoromethyl-4-oxo-4H-[1,3]thiazeto[3,2-a]quinoline-3-carboxylate obtained in the same manner as Example 4, 231 mg of 4-methylpiperazine, and 7 ml of DMF, the reaction was conducted at ambient temperature for 17 hours. To this reaction mixture was added 30 ml of ether and the resulting crystals were collected by filtration and dissolved in chloroform. This solution was washed with water, dried over magnesium sulfate, and finally concentrated.
m. p. 232°C
Specific rotation [α]²⁰ = +67.60° (DMF, c = 0.707)

| Elemental analysis for C₁₉H₂₁F₂N₃O₃S | | | |
|---|---|---|---|
| Calcd. (%): | C, 55.73; | H, 5.17; | N, 10.26 |
| Found (%): | C, 55.34; | H, 5.09; | N, 9.90 |

### Example 58

### Production of ethyl (-)-6-fluoro-1-fluoromethyl-7-(4-methyl-1-piperazinyl)-4-oxo-4H-[1,3]thiazeto[3,2-a]quinoline-3-carboxylate (synthesis of optical isomer)

Using the (-)-compound obtained in Production Example 4, the desirable compound was synthesized in the same manner as Example 57.
m. p. 238-240°C
Specific rotation [α]²⁰ = -82.59° (DMF, c = 1.00)

| Elemental analysis for C₁₉H₂₁F₂N₃O₃S | | | |
|---|---|---|---|
| Calcd. (%): | C, 55.73; | H, 5.17; | N, 10.26 |
| Found (%): | C, 55.79; | H, 4.95; | N, 10.10 |

### Example 59

### Production of an optical isomer of (+)-6-fluoro-1-fluoromethyl-7-(4-methyl-1-piperazinyl)-4-oxo-4H-[1,3]thiazeto[3,2-a]quinoline-3-carboxylic acid hemisulfate

A mixture of 250 mg of the (+)-ester obtained in Example 57 and 2.5 ml of fuming sulfuric acid was allowed to react under ice-cooling for 19 hours. The reaction mixture was then poured in 30 ml of iced water and the resulting crystals were collected by filtration. This crystals were washed with water, ethanol and ether and dried to provide 195 mg of colorless powder.
m. p. 262°C (decomp.)

| Elemental analysis for C₁₇H₁₇F₂N₃O₃S·1/2H₂SO₄·3/2H₂O | | | |
|---|---|---|---|
| Calcd. (%): | C, 44.63; | H, 4.63; | N, 9.19 |
| Found (%): | C, 44.30; | H, 4.24; | N, 9.20 |

Specific rotation [α]²⁰ = + 73.38° (DMSO, c = 0.278)

### Example 60

### Production of an optical isomer of (-)-6-fluoro-1-fluoromethyl-7-(4-methyl-1-piperazinyl)-4-oxo-4H-[1,3]thiazeto[3,2-a]quinoline-3-carboxylic acid hemisulfate

Using the compound obtained in Example 58, the procedure of Example 59 was otherwise repeated to provide the (-)-compound.
m. p. 260-261°C (decomp.)

| Elemental analysis for C₁₇H₁₇F₂N₃O₃S·1/2H₂SO₄·H₂O | | | |
|---|---|---|---|
| Calcd. (%): | C, 45.53; | H, 4.49; | N, 9.36 |
| Found (%): | C, 45.11; | H, 4.05; | N, 9.21 |

Specific rotation [α]²⁰ = -50.16° (DMSO, c = 0.307)
The following compounds can be obtained in the same manner as above.
6,8-Difluoro-1-methylene-4-oxo-7-(1-piperazinyl)-4H-[1,3]thiazeto[3,2-a]quinoline-3-carboxylic acid,
Ethyl 6,8-difluoro-1-fluoromethyl-4-oxo-7-(4-trifluoroacetylaminophenyl)-4H-[1,3]thiazeto[3,2-a]quinoline-3-carboxylate,
7-(4-Aminophenyl)-6,8-difluoro-1-methylene-4-oxo-4H-[1,3]thiazeto[3,2-a]quinoline-3-carboxylic acid,
Ethyl 6,8-difluoro-1-fluoromethyl-4-oxo-7-(4-trifluoroacetylaminomethyl-1-phenyl)-4H-[1,3]thiazeto[3,2-a]quinoline-3-carboxylate,
Ethyl 7-(3-amino-1-pyrrolidinyl)-6-fluoro-1-fluoromethyl-4-oxo-4H-[1,3]thiazeto[3,2-a]quinoline-3-carboxylate,
Ethyl (1S)(3S)-7-(3-amino-1-pyrrolidinyl)-6-fluoro-1-fluorometyl-4-oxo-4H-[1,3]thiazeto[3,2-a]quinoline-3-carboxylate,
Ethyl (1S)(3R)-7-(3-amino-1-pyrrolidinyl)-6-fluoro-1-fluoromethyl-4-oxo-4H-[1,3]thiazeto[3,2-a]quinoline-3-carboxylate,
Ethyl (1R)(3S)-7-(3-amino-1-pyrrolidinyl)-6-fluoro-1-fluoromethyl-4-oxo-4H-[1,3]thiazeto[3,2-a]quinoline-3-carboxylate,
Ethyl (1R)(3R)-7-(3-amino-1-pyrrolidinyl)-6-fluoro-1-fluoromethyl-4-oxo-4H-[1,3]thiazeto[3,2-a]quinoline-3-carboxylate.

Optical isomers of the following compounds can be produced in the same manner as above.
n-Butyl 6-fluoro-1-fluoromethyl-7-(4-methyl-1-piperazinyl)-4-oxo-4H-[1,3]thiazeto[3,2-a]quinoline-3-carboxylate
n-Amyl 6-fluoro-1-fluoromethyl-7-(4-methyl-1-piperazinyl)-4-oxo-4H-[1,3]thiazeto[3,2-a]quinoline-3-carboxylate
n-Octyl 6-fluoro-1-fluoromethyl-7-(4-methyl-1-piperazinyl)-4-oxo-4H-[1,3]thiazeto[3,2-a]quinoline-3-carboxylate
Below shown are pharmacological test data for some representative species of the compound of the invention, which indicate the usefulness of the compound.
1. Determination of minimal inhibitory concentration (MIC) Method: MIC values were determined by agar plate dilution assay in accordance with the standard method of Japan Society of Chemotherapy (Chemotherapy 29: 76-79 (1981)). Thus, a suspension of cells grown in the bouillon medium for sensitivity assay at 37°C for 18 hours was diluted with the same medium to a concentration of 10⁶ CFU/ml. Using a microplanter, this dilution was inoculated on a sensitivity assay agar medium containing the test drug and incubated at 37°C for 18 hours. The MIC value was then determined. As control drugs, ciprofloxacin (CPFX) and tosufloxacin (TFLX) were used. The results are shown in Tables 1-3.

**Table 1**

| Antibacterial activity against various bacteria | | | | |
|---|---|---|---|---|
| Strain | MIC (µg/ml) | | | |
| | Example 5 | Example 15 | CPFX | TFLX |
| Staphylococcus aureus 209-P JC-1 (1) | ≦ 0.006 | 0.012 | 0.10 | 0.025 |
| Staphylococcus aureus Smith (2) | ≦ 0.006 | ≦ 0.006 | 0.10 | ≦ 0.006 |
| Staphylococcus aureus OWPH 1984 (MRSA) (3) | ≦ 0.006 | ≦ 0.006 | 0.39 | 0.025 |
| Staphylococcus aureus OWPH 2125 (MRSA, PCA^{r}) (4) | 0.012 | 0.025 | 12.50 | 0.78 |
| Staphylococcus epidermidis IFO 12993 (5) | ≦ 0.006 | 0.012 | 0.39 | 0.10 |
| Enterococcus faecalis ATCC 29212 (6) | 0.025 | 0.025 | 0.39 | 0.10 |
| Bacillus subtilis ATCC 6633 (7) | ≦ 0.006 | ≦ 0.006 | 0.05 | 0.012 |
| Escherichia coli KC-14 (8) | 0.025 | 0.05 | 0.012 | 0.012 |
| Pseudomonas aeruginosa IFO 3445 (9) | 0.10 | 0.20 | 0.20 | 0.39 |
| MRSA; Methicillin-resistant Staphylococcus aureus PCA^{r}; New quinolone-resistant strain | | | | |

**Table 2**

| Antibacterial activity against various bacteria | | | | | | |
|---|---|---|---|---|---|---|
| Strain | MIC (µg/ml) | | | | | |
| | Example 18 | Example 23 | Example 26 | Example 29 | Example 33 | Example 38 |
| (1) | 0.10 | 0.20 | 0.012 | 0.10 | 0.10 | 0.05 |
| (2) | 0.05 | 0.20 | 0.012 | 0.025 | 0.10 | 0.10 |
| (3) | 0.10 | 0.39 | 0.025 | 0.20 | 0.10 | 0.39 |
| (4) | 0.39 | 3.13 | 0.20 | 1.56 | 0.39 | 12.5 |
| (5) | 0.10 | 0.78 | 0.05 | 0.20 | 0.20 | 0.39 |
| (6) | 0.39 | 1.56 | 0.10 | 0.20 | 0.78 | 0.78 |
| (7) | 0.05 | 0.10 | 0.012 | 0.10 | 0.05 | 0.20 |
| (8) | 0.025 | 0.05 | ≦0.006 | 0.025 | 0.05 | 0.025 |
| (9) | 0.78 | 0.78 | 0.05 | 0.39 | 1.56 | 0.39 |

**Table 3**

| Antibacterial activity against various bacteria | | | |
|---|---|---|---|
| Strain | MIC (µg/ml) | | |
| | Example 41 | Example 59 | Example 60 |
| (1) | 0.012 | 0.39 | 0.025 |
| (2) | ≦0.006 | 0.20 | 0.025 |
| (3) | 0.025 | 0.39 | 0.10 |
| (4) | 1.56 | 12.5 | 0.78 |
| (5) | 0.05 | 0.78 | 0.20 |
| (6) | 0.78 | 3.13 | 0.20 |
| (7) | ≦0.006 | 0.20 | 0.05 |
| (8) | 0.05 | 0.10 | 0.012 |
| (9) | 1.56 | 0.78 | 0.20 |

2. Therapeutic efficacy against infections in mice Method: In 5% mucin was suspended 2.5 x 10⁶ CFU/mouse of Staphylococcus aureus Smith, 5.0 x 10⁶ CFU/mouse of quinolone-resistant and methicillin-resistant S. aureus KC 5879, or 2.5 x 10⁵ CFU/mouse of Pseudomonas aeruginosa E-2, and male ddY mice (body weights 20 g, approx., 4 weeks old; 5-7 individuals per group) were inoculated intraperitoneally with the resulting suspension. The drug was administered orally 2 hours after inoculation and the ED₅₀ was calculated from the survival rate after one week by probit analysis. As a reference control drug, ciprofloxacin was used. The results are shown in Tables 4 and 5.

**Table 4**

| Strain | ED₅₀ (mg/kg) | | | | |
|---|---|---|---|---|---|
| | Example 12 | Example 17 | Example 18 | Example 22 | Example 23 |
| Staphylococcus aureus Smith (1) | 2.2 | 2.02 | 3.54 | 3.3 | 2.8 |
| Staphylococcus aureus KC 5879* (2) | 34.9 | 18.3 | - | 42.1 | - |
| Pseudomonas aeruginosa E-2 (3) | 20.7 | 22.2 | - | 25.2 | >50 |

| | | | | | |
|---|---|---|---|---|---|
| * : Quinolone-resistant and methicillin-resistant Staphylococcus aureus | | | | | |

**Table 5**

| Strain | ED₅₀ (mg/kg) | | | | | |
|---|---|---|---|---|---|---|
| | Example 29 | Example 33 | Example 36 | Example 59 | Example 60 | CPFX |
| (1) | 2.43 | 2.9 | 2.4 | 5.44 | 1.00 | 11.00 |
| (2) | 38.8 | 39.9 | 51.1 | - | - | >200 |
| (3) | 23.3 | 42.7 | 32.2 | 24.72 | 27.32 | 46.55 |

### EFFECT OF THE INVENTION

The compound of the present invention is effective in the treatment of various infections, particularly the infections caused by gram-positive bacteria including methicillin-resistant strains and new quinolone-resistant and methicillin-resistant strains of Staphylococcus aureus.

## Claims

1. A quinolinecarboxylic acid derivative of the following general formula [I] or a pharmaceutically acceptable salt thereof. 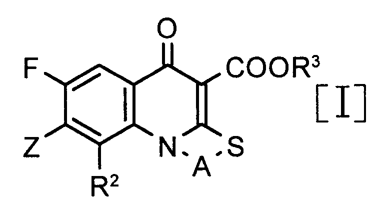 wherein Z represents substituted or unsubstituted cyclic amino or phenyl;
A represents >C=CH₂ or >CHR¹ (R¹ : fluoroalkyl);
R² represents hydrogen, alkyl, alkoxy, amino, or halogen; and
R³ represents hydrogen or alkyl.

2. The compound of claim 1 wherein A represents >CHR¹ (R¹: fluoroalkyl); Z represents 4-substituted or unsubstituted piperazinyl; R² represents hydrogen, alkoxy, or halogen; R³ represents hydrogen or alkyl.

3. The compound of claim 1 wherein R¹ represents fluoromethyl; Z represents piperazinyl or 4-methylpiperazinyl; R² represents hydrogen, methoxy, fluorine or chlorine; R³ represents hydrogen or ethyl.

4. The compound of claim 1 wherein A represents >C=CH₂; Z represents substituted or unsubstituted pyrrolidinyl, piperazinyl or phenyl; R² represents hydrogen, alkoxy, or halogen; R³ represents hydrogen or alkyl.

5. The compound of claim 1 wherein A represents >C=CH₂; Z represents 3-aminopyrrolidinyl, pyrrolidinyl, 4-methylpiperazinyl, piperazinyl or amino-substituted phenyl; R² represents hydrogen, methoxy, fluorine, or chlorine; R³ represents hydrogen or ethyl.

6. The compound of claim 1 which is 6-fluoro-1-fluoromethyl-7-(4-methyl-1-piperazinyl)-4-oxo-4H-[1,3]thiazeto[3,2-a]quinoline-3-carboxylic acid.

7. The compound of claim 1 which is (-)-6-fluoro-1-fluoromethyl-7-(4-methyl-1-piperazinyl)-4-oxo-4H-[1,3]thiazeto[3,2-a]quinoline-3-carboxylic acid.

8. The compound of claim 1 which is 6-fluoro-1-methylene-7-(3-amino-1-pyrrolidinyl)-4-oxo-4H-[1,3]thiazeto[3,2-a]quinoline-3-carboxylic acid.

9. A quinolinecarboxylic acid derivative of the following general formula [XII] or a pharmaceutically acceptable salt thereof. 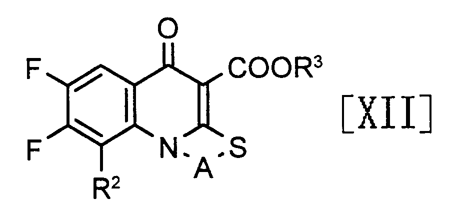 wherein A represents >C=CH₂ or >CHR¹ (R¹ : fluoroalkyl);
R² represents hydrogen, alkyl, alkoxy, amino or halogen;
R³ represents hydrogen or alkyl.

10. The compound of claim 9 wherein A represents >CHR¹ (R¹ : fluoroalkyl); R² represents hydrogen, alkyl, alkoxy, amino or halogen; R³ represents hydrogen or alkyl.

11. The compound of claim 9 wherein R¹ represents fluoromethyl; R² represents hydrogen, methoxy, fluorine or chlorine; R³ represents hydrogen or ethyl.

12. A quinolinecarboxylic acid derivative of the following general formula [XI] or a pharmaceutically acceptable salt thereof. 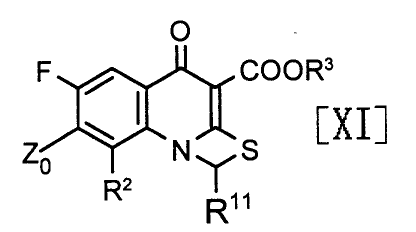 wherein Z₀ represents halogen, or substituted or unsubstituted cyclic amino or phenyl;
R² represents hydrogen, alkyl, alkoxy, amino, or halogen;
R³ represents hydrogen or alkyl;
R¹¹ represents hydroxyalkyl, acyloxyalkyl, or alkoxyalkyl.

13. A process for producing optically active quinolinecarboxylic acid derivatives of the following general formulas [IIf] and [IIg] characterized by subjecting a substrate of the following general formula [IIe] to stereoselective alcoholysis of one of its optical isomers in the presence of an enzyme having stereoselective transesterase activity followed by isolation. 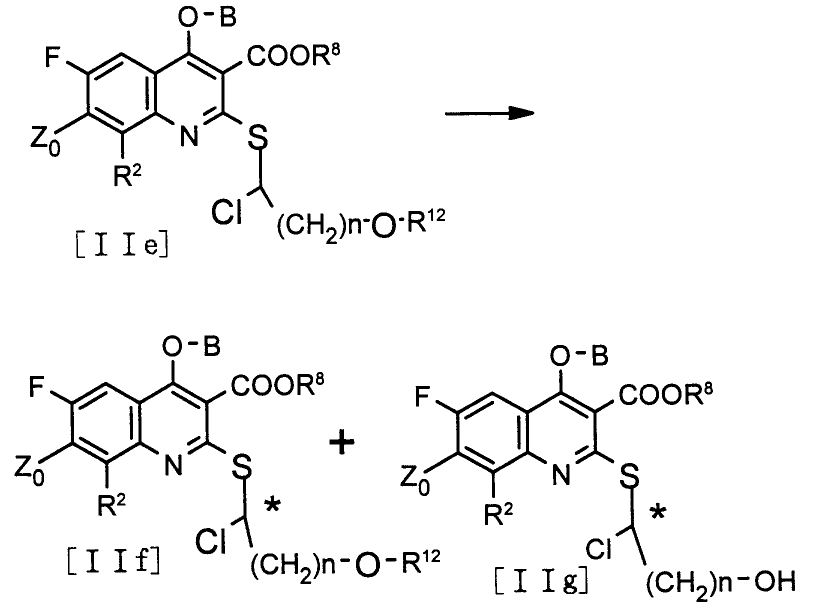 In the formulas, Z₀ represents halogen, or substituted or unsubstituted cyclic amino or phenyl;
R² represents hydrogen, alkyl, alkoxy, amino, or halogen;
R⁸ represents alkyl;
R¹² represents acyl.

14. A process for producing optically active thiazetoquinolinecarboxylic acid derivatives of the following general formulas [XIf] and [XIg] characterized by subjecting a substrate of general formula [XIe] to stereoselective alcoholysis of one of its optical isomers in the presence of an enzyme having stereoselective transesterase activity followed by isolation. 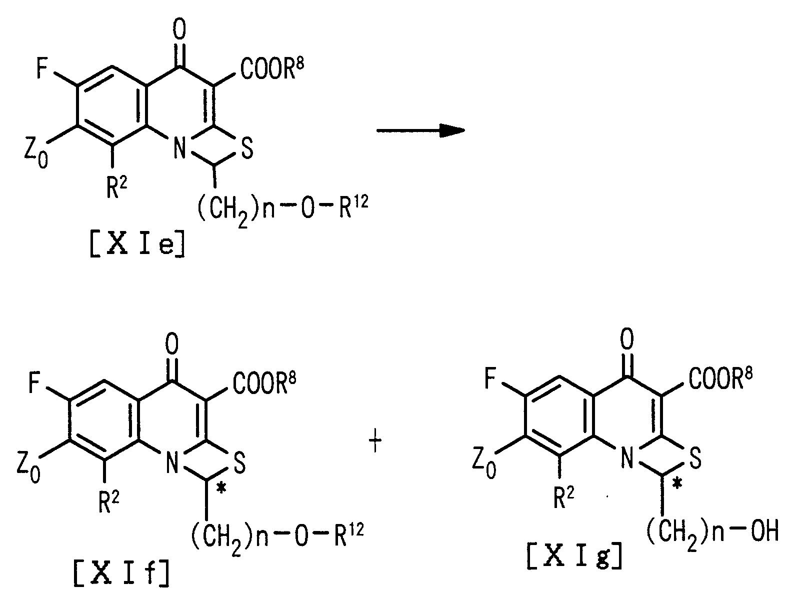 In the formulas, Z₀ represents halogen, or substituted or unsubstituted cyclic amino or phenyl;
R² represents hydrogen, alkyl, alkoxy, amino, or halogen;
R³ represents alkyl;
R¹² represents acyl.

15. A process for producing optically active thiazetoquinolinecarboxylic acid derivatives of the following general formulas [XIg] and [XIf] characterized in that a substrate of the following general formula [XIk] is subjected to selective reaction of one of its optical isomers with an acyl donor in the presence of an enzyme having stereoselective transesterase activity and the resulting acyl compound is separated from the unreacted compound. 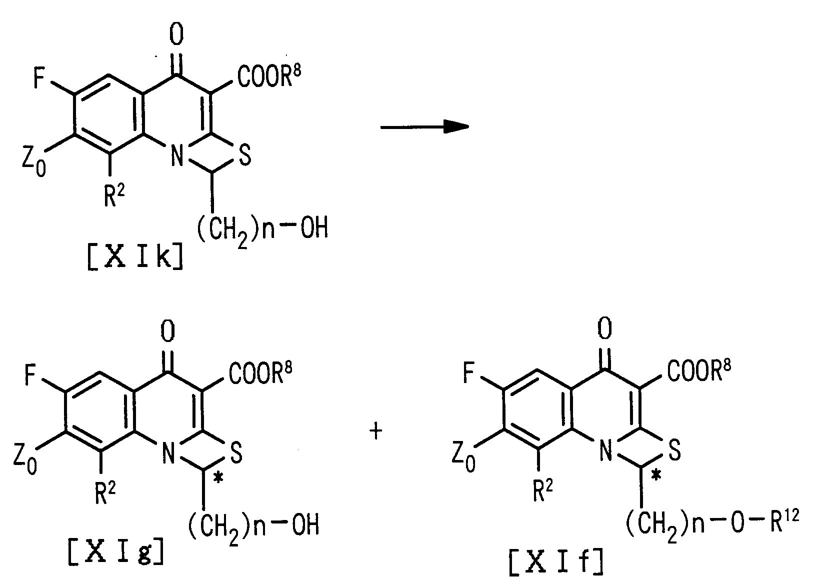 In the formulas, Z₀ represents halogen, or substituted or unsubstituted cyclic amino or phenyl;
R² represents hydrogen, alkyl, alkoxy, amino, or halogen;
R³ represents alkyl;
R¹² represents acyl.
